(19) Europäisches Patentamt
European Patent Office
Office européen des brevets

(11) Publication number : **0 390 762 B1**

# (12) EUROPEAN PATENT SPECIFICATION

(45) Date of publication of patent specification : **19.01.94 Bulletin 94/03**

(51) Int. Cl.[5] : **C07C 215/60,** C07C 215/76, C07C 217/60, C07D 317/58, A61K 31/135, A61K 31/335

(21) Application number : **90850112.5**

(22) Date of filing : **23.03.90**

## (54) New bronchospasmolytic compounds and process for their preparation.

The file contains technical information submitted after the application was filed and not included in this specification

(30) Priority : **28.03.89 SE 8901060**

(43) Date of publication of application : **03.10.90 Bulletin 90/40**

(45) Publication of the grant of the patent : **19.01.94 Bulletin 94/03**

(84) Designated Contracting States : **AT BE CH DE DK ES FR GB GR IT LI LU NL SE**

(56) References cited :
**US-A- 3 700 692**
**US-A- 4 072 760**

(73) Proprietor : **Aktiebolaget Draco**
**Box 34**
**S-221 00 Lund (SE)**

(72) Inventor : **Olsson, Otto Agne Torsten**
**Hjortgatan 3**
**S-223 50 Lund (SE)**
Inventor : **Pedaja, Peter Juhan**
**Spexarvägen 36 B**
**S-223 71 Lund (SE)**
Inventor : **Svensson, Leif Ake**
**Hövdingavägen 39**
**S-223 75 Lund (SE)**
Inventor : **Waldeck, Carl Bertil**
**Sofielundsvägen 34**
**S-240 17 S.Sandby (SE)**
Inventor : **Wetterlin, Kjell Ingvar Leopold**
**Västervang 19**
**S-240 17 S.Sandby (SE)**

(74) Representative : **Linderoth, Margareta et al**
**AB Astra Patent Department**
**S-151 85 Södertälje (SE)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

Field of the invention

The present invention relates to novel compounds having therapeutic activity, to processes for their preparation, to pharmaceutical preparations containing them and to medicinal use of the compounds. In particular, the compounds of the invention have bronchospasmolytic effect and are effective in the treatment of reversible obstructive lung ailments of various genesis, particularly asthmatic conditions. The compounds, when possessing the proper stereochemical configuration, exhibit a prolonged duration of therapeutic effect after inhalation and a reduced degree of side effects, especially a reduced heart-stimulating effect.

Background of the invention

It is desirable to find bronchodilating agents which have longer duration of activity than the substances which are available on the market. A longer duration of activity will make it possible to give the asthmatic patient adequate protection also during the night and the early morning hours, when the asthma usually is at worst. The bronchodilator drugs currently used for inhalation therapy (e.g. fenoterol, salbutamol, terbutaline and bitolterol) generally have a duration of activity lasting for only 4-6 hours, and will therefore not give adequate protection during the night, if taken at bed time. Bitolterol is described by Kass, I. and Mingo, T.S. in Chest 1980, 78:283 "A new longacting bronchodilator with reduced chronotopic effects". US 4 072 760 describes compounds, which are closely related structurally to those of the present application, but only short acting.

US-A-3 700 692 dicloses 1-(4'-hydroxy-3'-(hydroxymethyl)-phenyl)-1-hydroxy-2-aralkylaminoethanes which are useful as bronchodilating agents.

Attempts to obtain bronchospasmolytically active compounds with long duration of activity are reported in the literature: e.g. formoterol, salmeterol. The problem was, however, to find bronchospasmolytically active compounds which have a clinically useful duration of activity of at least 12 hours following inhalation of an aerosol of micronized substance. It is also desirable that these compounds bind firmly to the lung tissue so that only minor amounts of the active compounds will leak into the general circulation. In this way the risk of inducing systemic side effects, such as palpitations and tremor, are minimized.

Outline of the invention

It has been found that the new compounds of the formula (X)

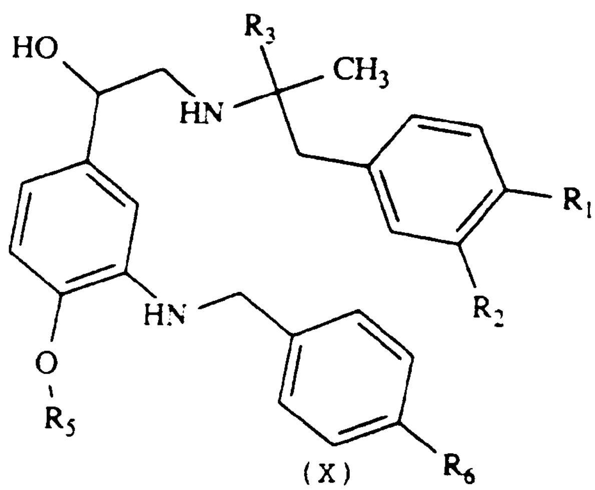

(X)

and pharmaceutically acceptable salts thereof, wherein

$R_1$ and $R_2$ are the same or different and each is H, OH, $OCH_3$, $OC_2H_5$, halogen or $R_1$ and $R_2$ together form the chain $-O-CH_2-O-$ or $-O-CF_2-O-$;

$R_3$ is H or $CH_3$;

$R_5$ is H or $-CO-R_7$;

$R_5$ is H, OH, $OCH_3$, $OC_2H_5$ or halogen;

$R_7$ is a straight or branched alkyl group with 1-16 carbon atoms, aryl, $-NH-CH_3$ or $N(CH_3)_2$;

are highly effective, and have a long duration of the bronchospasmolytic activity, especially when administered locally to the lungs, e.g. after inhalation of an aerosolized or nebulized formulation of the compounds.

In a compound of the above formula (X):

aryl is a group with 3-8 carbon atoms, preferably 6; and halogen is fluor, chlor or brom. Pharmaceutically acceptable salts of the invention are especially acid addition salts. They may conveniently be prepared by contacting the compounds which are capable of forming such salts with a suitable physiologically acceptable organic or inorganic acid. Examples of suitable organic acids are acetic acid, fumaric acid, citric acid, tartaric acid, maleic acid, succinic acid and benzoic acid. Examples of suitable inorganic acids are hydrochloric acid, hydrobromic acid, sulfuric acid and phosphoric acid.

The compounds of the invention also exhibit a lower degree of undesired cardiovascular side effects. Both the pharmacological activity and the duration of activity of the new compounds depend highly on the stereochemical configuration at the benzylic stereogenic carbon atom in the phenyl ethanolamine chain and on the stereogenic carbon atom in the substituent at the nitrogen. Thus, optimal pharmacological effect is obtained only with the R-configuration at the benzylic position, and duration is optimal with a S-configuration for the group, which is a substituent at the nitrogen.

Preferred compounds according to the invention are those, wherein $R_1$ and $R_2$ form the chain $-OCH_2-O-$ or $-O-CF_2-O-$;

$R_3$ is H or $CH_3$;

$R_5$ is H and

$R_5$ is $OCH_3$.

Especially preferred compounds according to the invention are: the racemic (R,S + S,R isomers) and the racemic (R,R + S,S isomers) of 1-[(4-hydroxy)-3-(p-methyoxybenzylamino)-phenyl]-2-[(1-metyl-2-(3,4-metylenedioxyphenyl)ethyl)amino]ethanol.

More especially preferred according to the invention is the isomer 1-(R)-(4-hydroxy)-3-(p-methoxybenzylamino)phenyl-2-[(1-(S)-methyl-2-(3,4-methylenedioxyphenyl)ethyl)amino]ethanol.

Preparation

The compounds of the formula (X) are prepared by reacting 4-benzyloxy-3-nitro-styreneoxide of the formula (V)

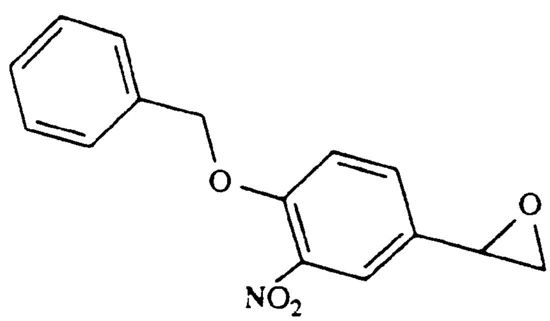

(V)

with a compound of the formula (VI)

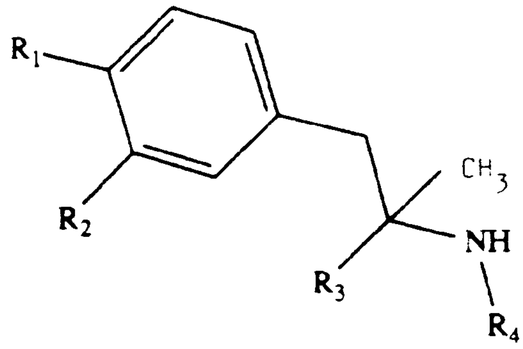

(VI)

wherein $R_1$, $R_2$ and $R_3$ are as defined above and $R_4$ is H or

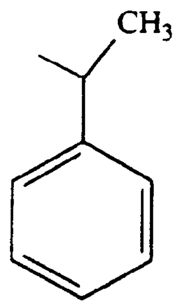

to give a compound of the formula (VII)

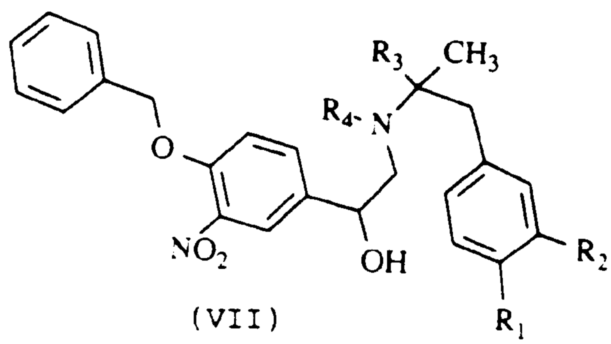

(VII)

wherein $R_1$, $R_2$, $R_3$ and $R_4$ are as defined above, whereafter (VII) is hydrogenated to give directly or via a compound of the formula (VIII)

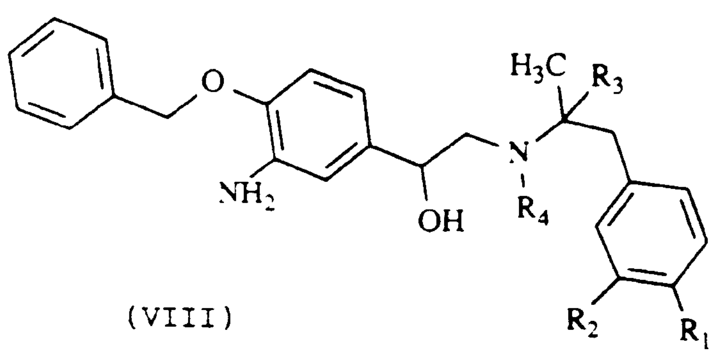

(VIII)

a compound of the formula (IX)

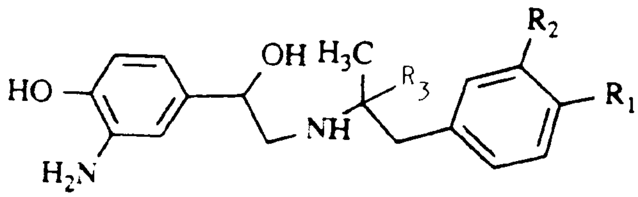

(IX)

wherein $R_1$, $R_2$ and $R_3$ are as defined above, whereafter the compound of the formula (IX) is reacted with the compound of the following formula

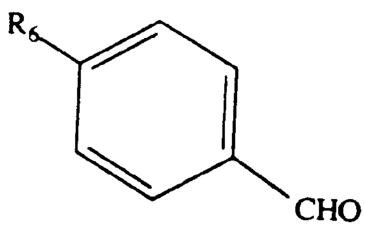

wherein $R_6$ is as defined above, together with $PtO_2$ or Pd on carbon and hydrogen, to yield a compound of the formula (X), where $R_5$ is H and $R_1$, $R_2$, $R_3$ and $R_6$ are as defined above, either as a pure isomer or as a mixture of various amounts of the possible stereo-isomeres, as described below.

General method of preparation

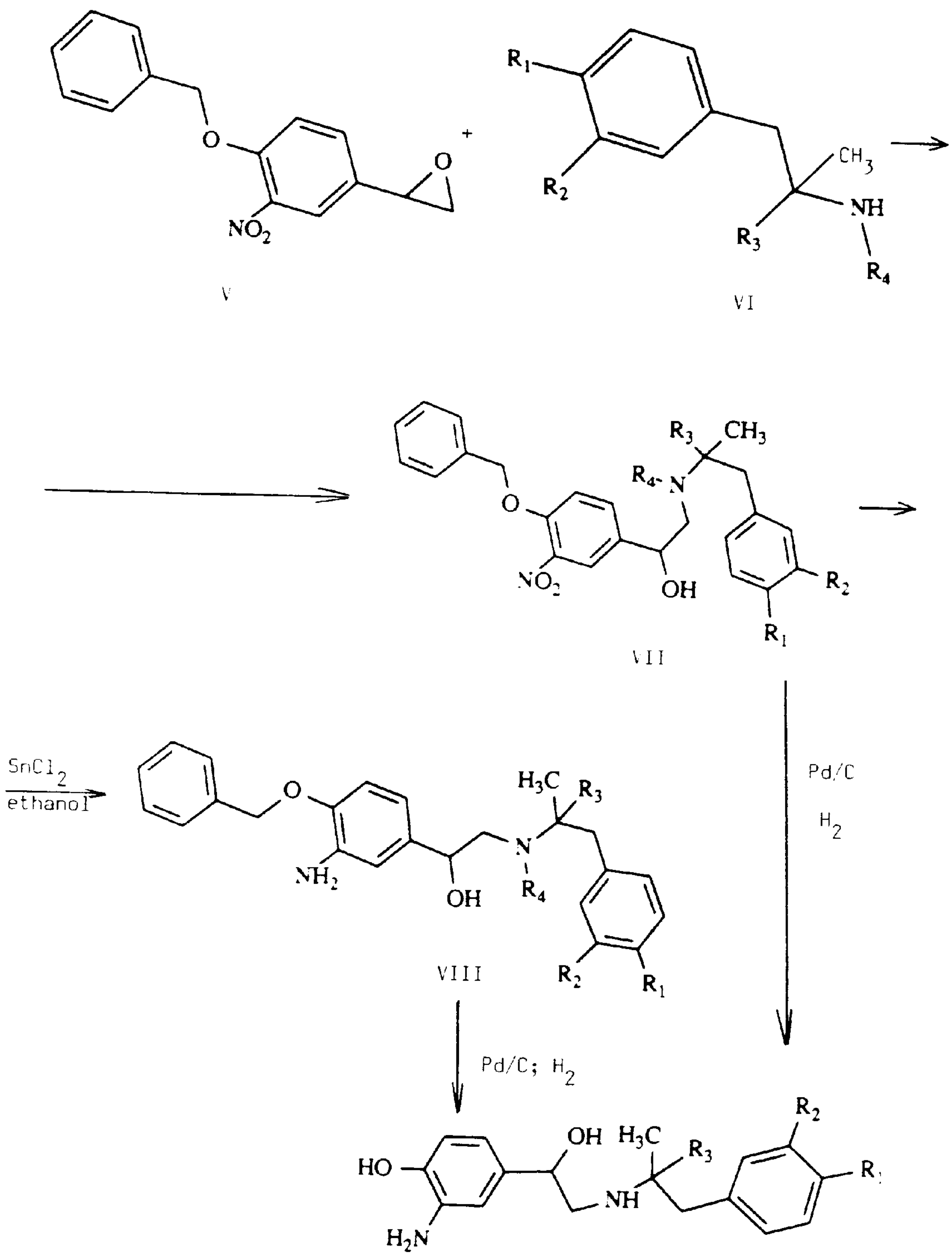


V

VI

VII

VIII

IX

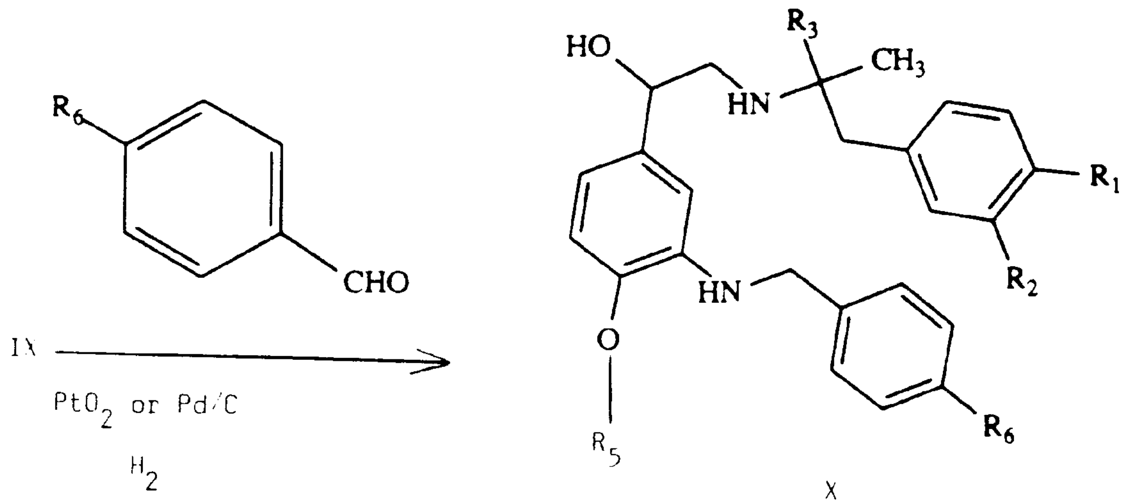


X

($R_5$ = H)

Method A

This method is used for the preparation of pure stereoisomers and epimers differing in configuration only at the benzylic position. ($R_1$, $R_2$ and $R_6$ are as defined above.)

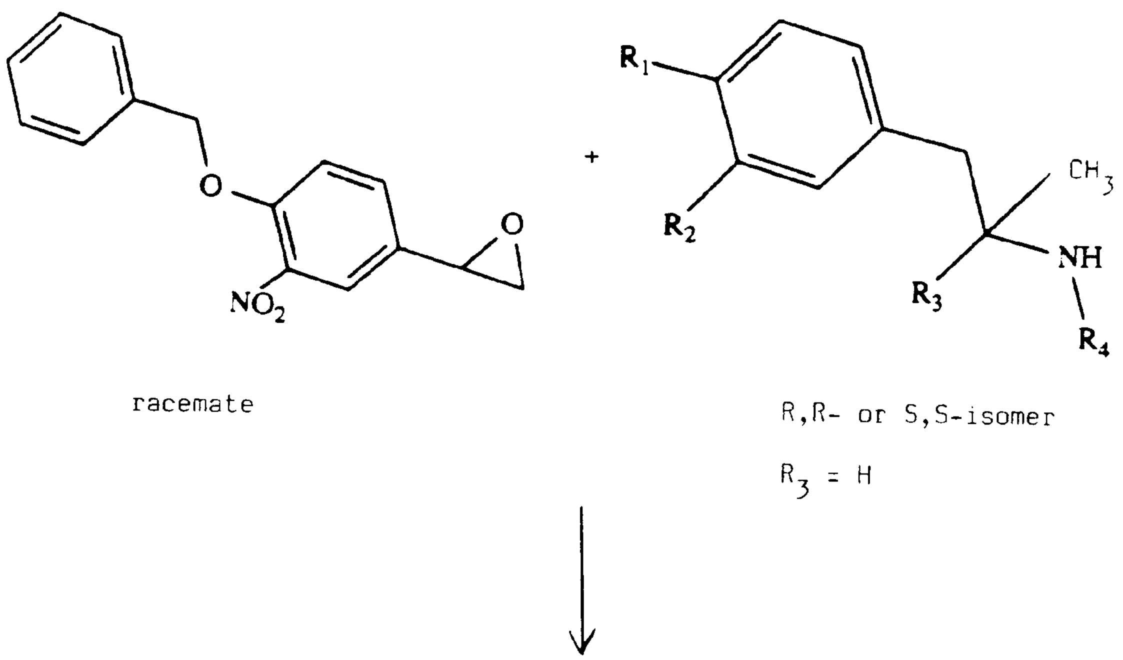

racemate

R,R- or S,S-isomer

$R_3$ = H

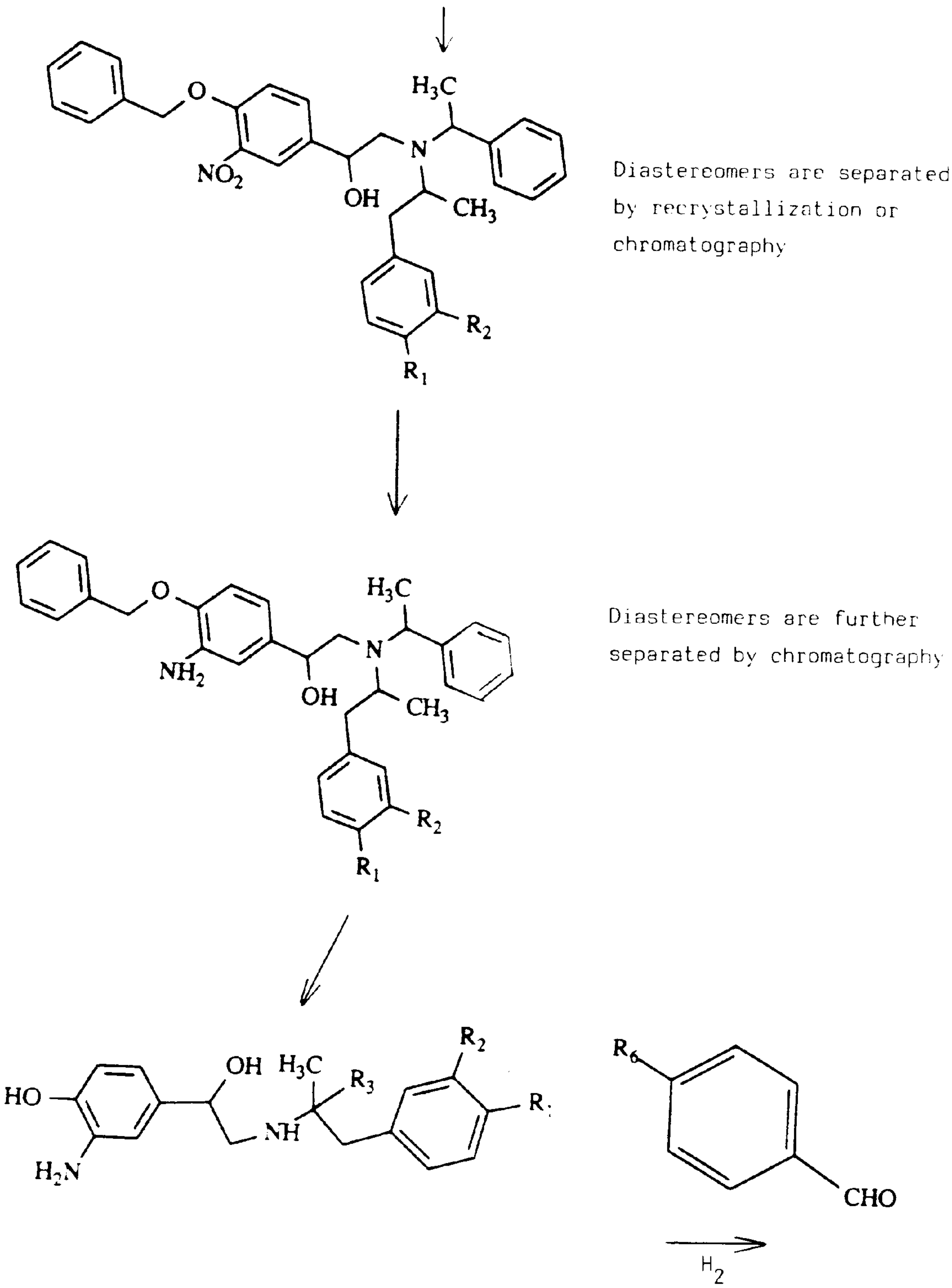
Diastereomers are separated by recrystallization or chromatography
Diastereomers are further separated by chromatography
H2

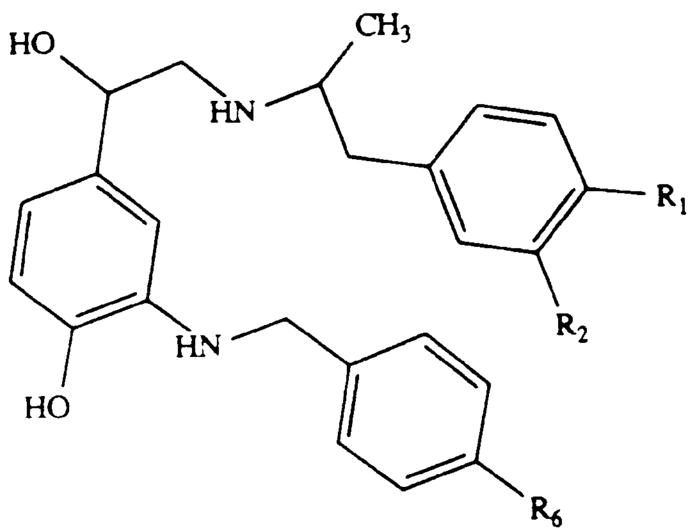

Method B

This method is used for the preparation of racemates and mixtures of racemates. ($R_1$, $R_2$, $R_3$ and $R_6$ are as defined above.)

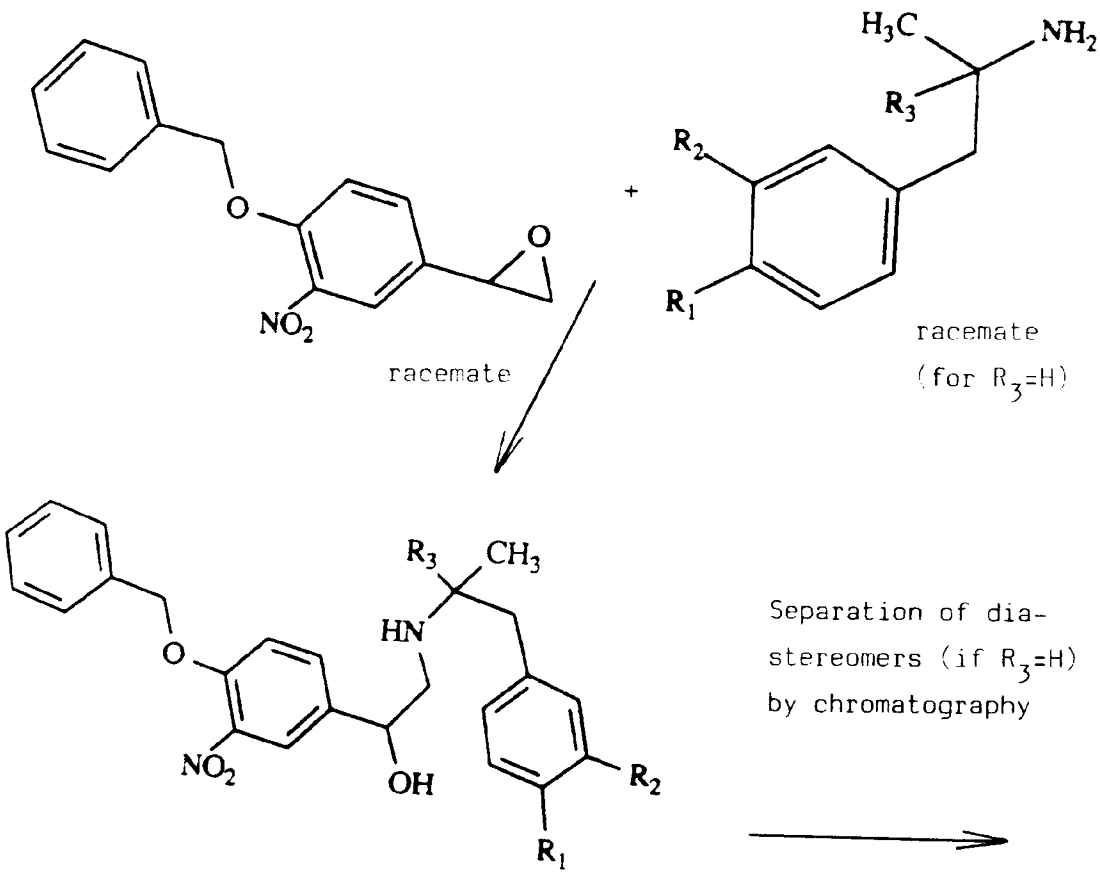

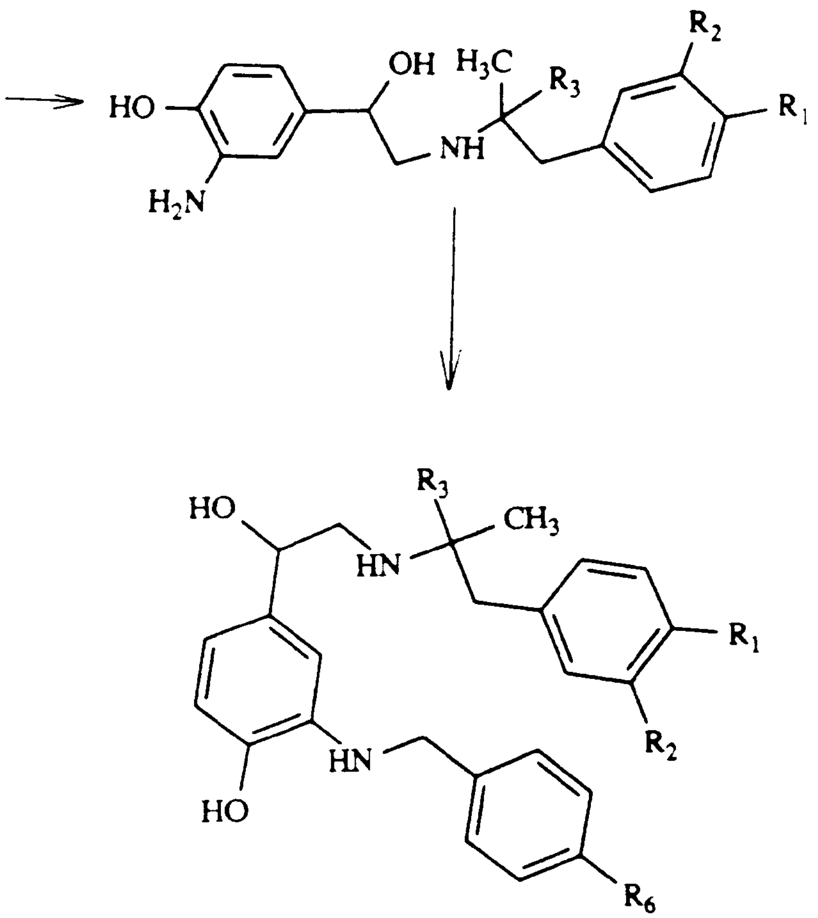

Preparation of starting compounds

Amines VI

Method C

Preparation of pure entantiomers

Suitably substituted 1-phenyl-2-propanones were reacted with R-or S-methyl-benzyl-amine followed by reduction of the imine by Rayney-Nickel giving the pure R,R-or S,S-amine, respectively.

$R_1$ and $R_2$ are as defined above.

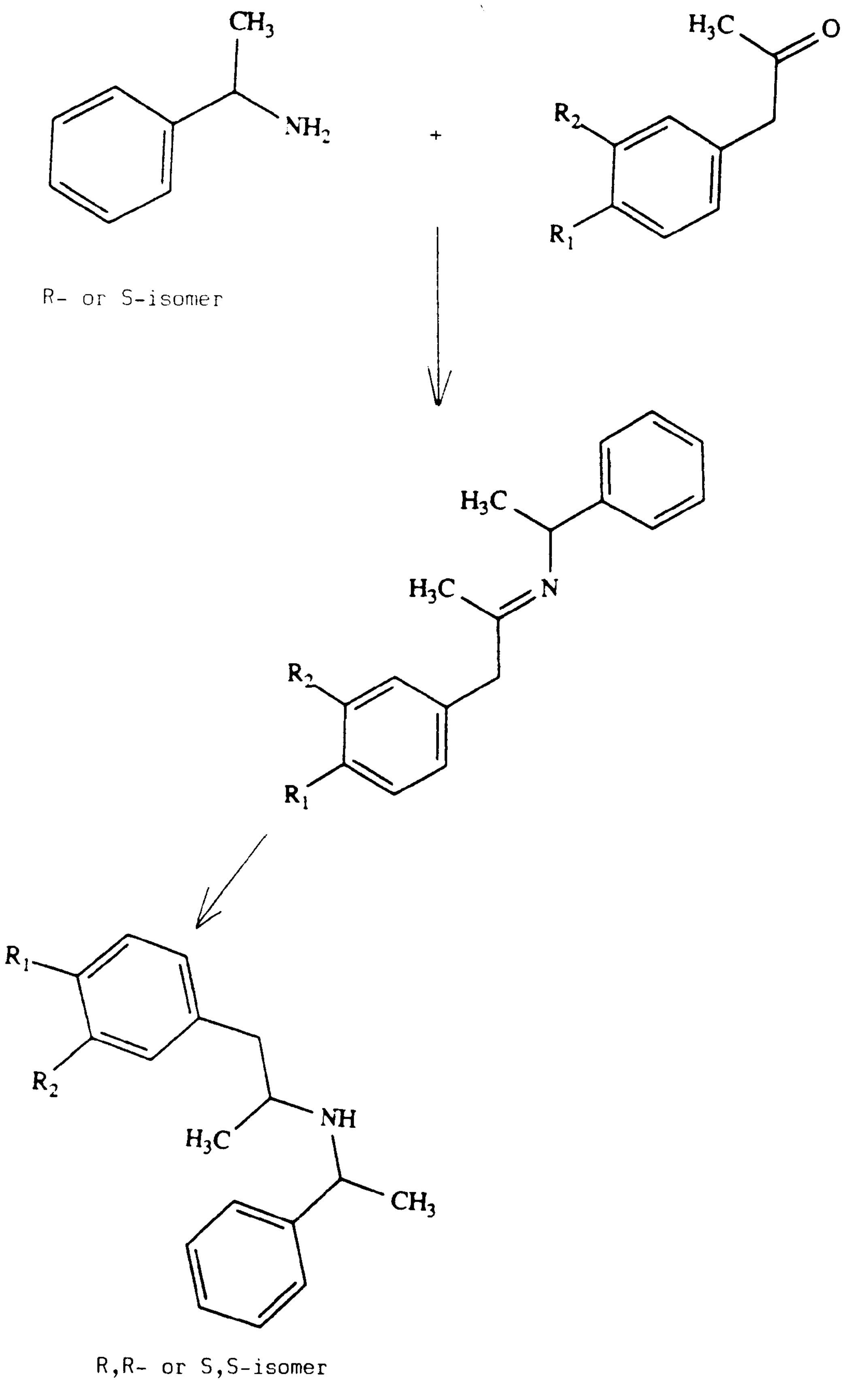
R- or S-isomer
R,R- or S,S-isomer

Method D

Preparation of racemic amines VI

Suitably substituted benzaldehydes were reacted with a nitroalkane followed by reduction to give the amine.

$R_1$, $R_2$ and $R_3$ are as defined above.

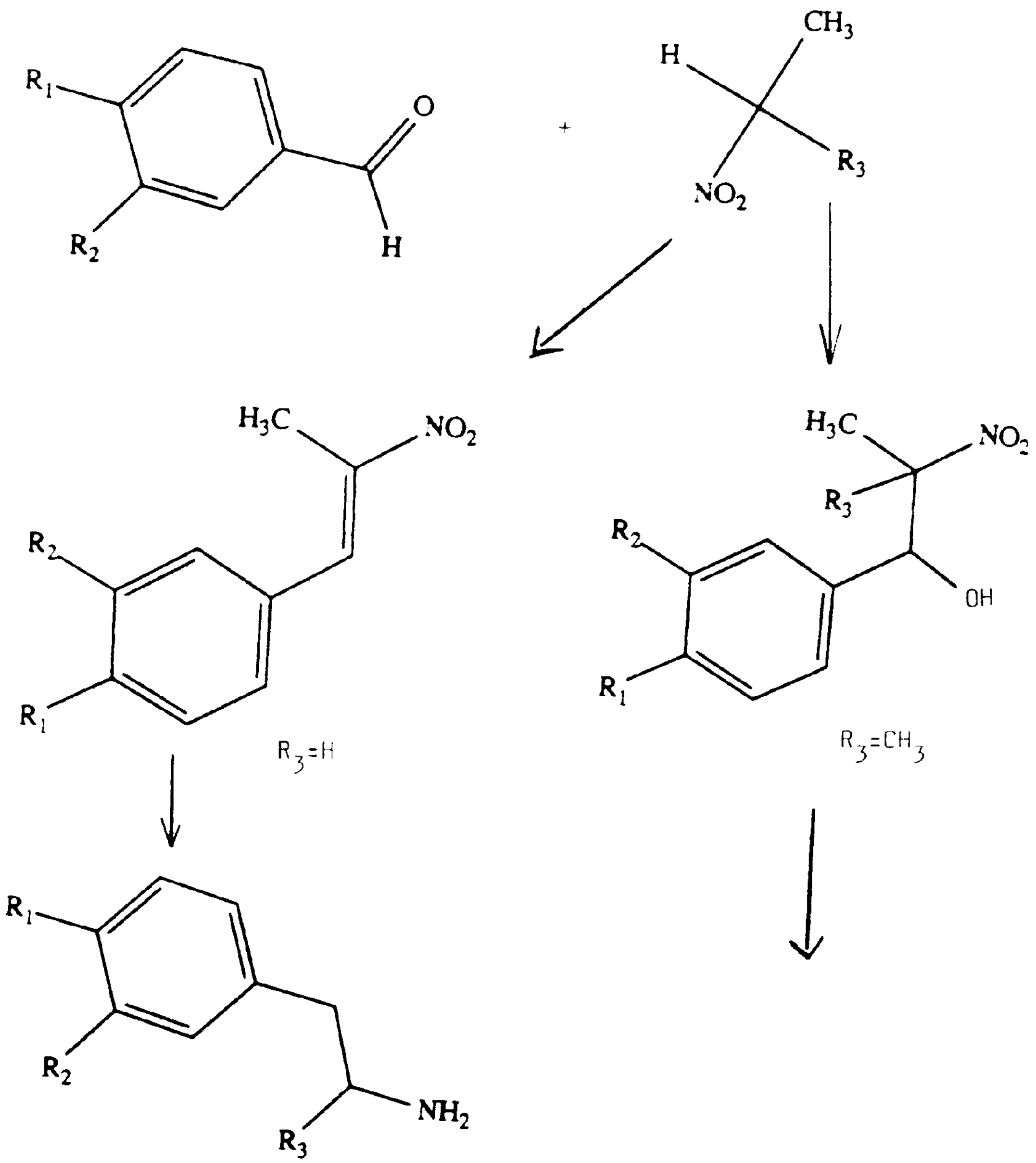

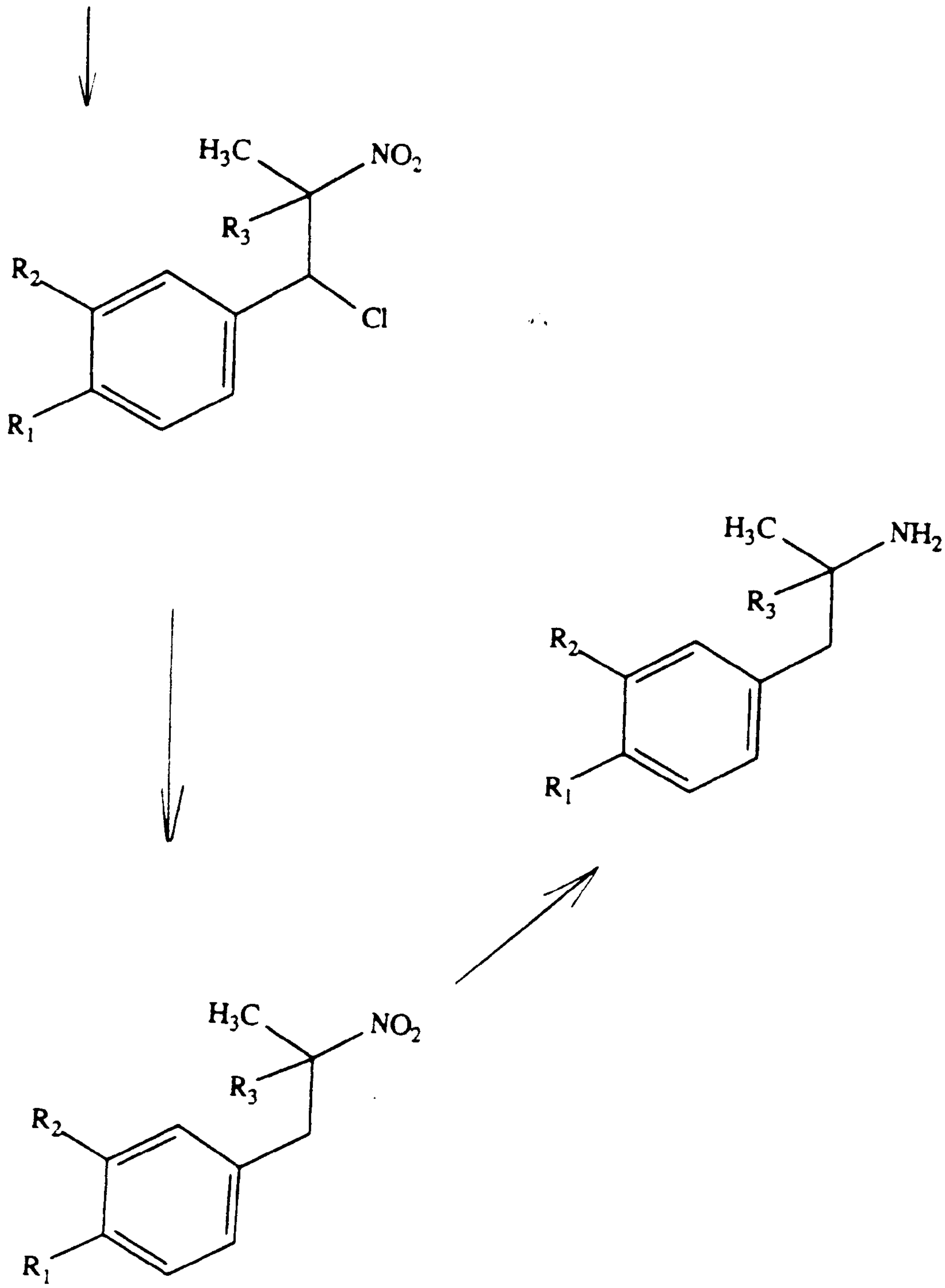

Epoxide

Racemic 4-benzyloxy-3-nitrostyrene oxide is prepared according to the method described in J. Med. Chem. 17, 49 (1974) by Kaiseo et al.

EXAMPLES

Example 1 (Preparation according to method A)

1-(R)-(4-hydroxy-3-(p-methoxybenzylamino)phenyl)-2-((1-(S)-methyl-2-(3,4-methylenedioxyphenyl)ethyl) amino)-ethanol (X)

1-(4-benzyloxy-3-nitrophenyl)-2-(N-methylbenzyl-1-methyl-2-(3,4-methylenedioxyphenyl))ethylamino-ethanol (VII) (R,S,S- and S,S,S-isomers)

23.9 g (88 mmol) of 4-benzyloxy-3-nitro-styreneoxide(V) and 24.9 g (88 mmol) of (S,S)-N-1-phenethyl-1-(1,3-benzodioxol-5-yl)-2-propanamine (VI) were stirred at 150°C under nitrogen for 15 h. The crude product was dissolved in ethyl acetate, conc. hydrochloric acid added and the solvent evaporated to give the crude hydrochloride as a solid. Recrystallization from ethyl acetate gave 17.3 g of the S,S,S- and R,S,S-isomer (85/15). Concentration of the mother liquor gave 29 g of the crude R,S,S and S,S,S-isomers (70/30). Flash chromatography of the concentrated mother liquor (petroleum ether/ethyl acetate (2.5/1), silica) gave 21.3 g of the 70/30 isomer mixture. The pure isomers R,S,S and S,S,S respectively were obtained by repeated flash chromatography or by chromatography with MeCN/silica.

NMR; (R,S,S isomer, $CDCl_3$);

7.85(d,1H), 7.54-6.33(m, 15H), 5.87(m, 2H), 5.23(s, 2H), 4.58(dd, 1H), 4.08(g, 1H), 3.06-2.54(m, 4H), 2.09(dd, 1H), 1.50(d, 3H), 0.96(d, 1H).

(S,S,S-isomer);

7.79(d, 1H), 7.47-6.28(m, 15H), 5.89(dd, 2H), 5.23(s, 2 H), 4.40(dd, 1H), 4.08(q, 1H), 3.18(m, 1H, 2.84-2.39(m, 4H), 1.44(d, 3H), 1.01(d, 3H).

The R,R,R- and S,R,R-isomers were prepared accordingly.

1-(4-benzyloxy-3-aminophenyl)-2-(N-methylbenzyl-1-methyl-2-(3,4-methylenedioxyphenyl))ethylamino-ethanol (VIII) (R,S,S- and S,S,S-isomers)

10.8 g (20 mmol) of the corresponding nitro compound (R,S,S,/S,S,S ratio 70/30) was dissolved in 250 ml of methanol. 23 g (100 mmol) of $SnCl_2;2H_2O$ was added and the solution heated to 60°C. During 50 min 400 mg of $NaBH_4$ was added portionwise. After stirring for additional 40 min at 60°C, the solution was concentrated to 150 ml and then 350 ml of 0.5 N aqueous NaOH was added followed by 200 ml of diethylether. Extraction of the aqueous phase with more diethylether, drying and evaporation, gave 7.9 g of an oily product. Chromatography (MeCN/silica) gave 4.5 g of the pure R,S,S isomer and 1.5 g of the S,S,S isomer.

NMR (R,S,S isomer, $CDCl_3$);

7.46-6.33(m, 16H), 5.87(d, 2H), 5.09(s, 2H), 4.54(dd, 1H), 4.08(q, 1H), 3.05-2.02(m, 5H), 1.50(d, 3H), 0.97(d, 3H).

(S,S,S-isomer);

7.43-6.25(m, 16H), 5.85(d, 2H), 5.05(s, 2H), 4.38(dd, 1H) ,4.08(q, 1H), 3.11(m, 1H), 2.81-2.36(m, 4H), 1.41(d, 3H), 0.95(d, 3H).

The monohydrochloride of the R,S,S-isomer was prepared by reacting 0.58 ml of acetyl chloride with methanol (180 ml) followed by addition of the base (4.5 g) and evaporation. After washing with diethylether, 4.3 g of the monohydrochloride was obtained as a salt. The S,R,R- and R,R,R-isomers were prepared accordingly.

(R)-1-(3-amino-4-hydroxyphenyl)-2-[(S)-1-methyl-2-(3,4-methylenedioxyphenyl)ethylamino]ethanol (IX)

4.3 g (8 mmol) of the previous compound (R,S,S,-isomer) - as hydrochloride -was dissolved in 75 ml of methanol. 0.3 g of 10% Pd/C was added and the mixture hydrogenated at 40 psi for 5 h. The methanol solution was filtered and this solution was used in the next step. The S,S,,- R,R, and S,R,-isomers of (IX) were prepared in a similar way.

1-(R)-(4-hydroxy-3-(p-methoxybenzylamino)phenyl)-2-[(1(S)-methyl-2-(3,4-methylenedioxyphenyl)ethyl)amino]ethanol (X)

2 ml (16 mmol) of p-anisaldehyde was added to the previously obtained methanol solution of the R,S,-isomer of (IX). After reflux for 30 min, 30 mg of $PtO_2$ was added and the mixture hydrogenated at 50 psi for 1.5 hrs. Yield of crude product (90% pure) was 2.3 g. Chromatography ($CHCl_3$/silica) of the base and addition of $H_2SO_4$/EtOH gave 1.2 g of pure (IV) as its sulfate.

NMR (R,S-isomer) DMSO,;

7.3-6.40(m, 10H), 5.99(s,2H), 4.52(d, 1H), 4.23(s, 2H), 3.71(s, 3H), 3.08(bs, 1H), 2.89-2.44(m and DMSO), 0.99(d, 3H).

Similarly prepared were the S,S- S,R- and R,R-isomers of (X).

NMR (S,S-isomer);

7.31-6.42(m, 10H), 5.98(s, 2H), 4.53(m, 1H), 4.26(s, 2H), 3.74(s, 3H), 3.1(m, 1H), 2.90-2.45(m and DMSO), 1.02(d, 3H).

Example 2 (Preparation according to method B)

Preparation of the racemates (R,S+S,R isomers) of 1-(4-hydroxy-3-(p-methoxybenzylamino)phenyl-2-[(1-methyl-2-(3,4-methylenedioxyphenyl)ethyl)amino]ethanol.

1-(4-benzyloxy-3-nitrophenyl-2-((1-methyl-2-(3,4-methylenedioxyphenyl))ethyl)amino-ethanol (VII) (various

mixtures of the R,R- R,S-, S,S- and S,R-isomers)

1.4 g (5 mmol) of (R/S)-4-benzyloxy-3-nitrostyrene oxide and 1.1 g (6 mmol) of (R/S)-1-(3,4-methylenedioxyphenyl)-2-amino-propane (VI) were refluexed in 10 ml of methanol for 20 h. After evaporation of the methanol, the crude isomeric mixture was dissolved in diethyl ether and the R,S- and S,R-isomers crystallized giving 0.4 g of the racemate. (The R,R and S,S isomers were removed.)

NMR ($CDCl_3$);

7.8(d, 1H), 7,5-6.5(m, 10H), 5.9(s,2H), 5.2(s,2H), 4.6(dd, 1H), 3.0-2.5(m, 5H), 1.2(d, 3H).

The racemate was hydrogenated (Pd/C in EtOH) to give the corresponding aminophenol (IX). This was then reacted with p-anisaldehyde as previously described, giving the racemic (R,S- + S,R-isomers) of the previously described compound (X). The R,R- + S,S-isomeric mixture was prepared from the previously removed isomeric mixture of (VII).

General procedure for the preparation of compounds of the formula (X)

The hydrochloride of the corresponding nitrocompound (VII) was dissolved in ethanol. 10% Pd/C was added and hydrogenation was carried out at room temperature for 4-20 hours at 50 psi. The catalyst was filtered off and benzaldehyde or p-methoxybenzaldehyde was added. After refluxing for 1 hour $PtO_2$ was added and hydrogenation made at 1 atm for 5-24 hours or at 50 psi for 45 min. The crude hydrochloride was washed with ether, the base taken up in ethylacetate and purified by chromatography on a silica column with chloroform/methanol as eluent (10/2). The pure base was then transformed to the hydrochloride or sulphate.

Example 3

1-(4-hydroxy-3-benzylamino-phenyl-2-(1-methyl-2-(3,4-methylenedioxyphenyl)-ethylamino)-ethanol (X)

(R,S+S,R+R,R+S,S mixture)

From 2.4 g of nitrocompound VII 0.7 g of Pd/C, 1.1 g of benzaldehyde and 40 mg of $PtO_2$, 1.2 g of the hydrochloride was obtained.

NMR: (DMSO-$D_6$ as solvent and reference) d; 7.4-6.4 (m,11H), 6.01 (s,2H), 5.90(d,1H), 5.35 (bs,1H), 4.72 (m,1H), 4.35 (s,2H), 3.37 (s,3H), 3.1-2.9 (m,4H), 1.10(d.3H).

Example 4

1-(4-hydroxy-3-p-methoxybenzylamino-phenyl)-2-(1-methyl-2-(3,4-dimethoxyphenyl)-ethylamino)-ethanol (X)

(R,S+S,S mixture)

From 5.2 g of nitrocompound VII, 0.5 g Pd/C, 3.0 g of p-methoxybenzaldehyde and 50 mg of $PtO_2$, 1.0 g of the product as hydrochloride was obtained.

NMR: (as above) d; 7.28 (d,2H), 7.0-6.5 (m,8H), 4.6 (m,1H), 4.22 (2H), 3.74(9H), 3.7-2.8(m ), 1.2 (d,3H).

Example 5

1-(4-hydroxy-3-p-methoxybenzylamino-phenyl)-2-(1,1-dimethyl-2-(3,4-methylenedioxyphenyl)-ethylamino)-ethanol (X)

(R/S racemate)

From 5.0 g of nitrocompound VII, 0.3 g of Pd/C, 4.9 ml of p-methoxybenzaldehyde and 30 mg of $PtO_2$, 3.1 g of the product as hydrochloride was obtained.

NMR: (as above) d; 7.3(d,2H), 6.9-6.4 (m,8H), 6.0(s,2H), 5.93 (m,1H), 5.24 (bs,1H), 4.73 (bd,1H), 4.25 (s,2H), 3.70 (s,3H), 3.37 (s,2H), 2.89(bm and s,5H), 1.19 (s,3H).

Example 6

1-(4-hydroxy-3-p-methoxybenzylamino-phenyl)-2-(1-methyl-2-(p-methoxyphenyl)-ethylamino)-ethanol (X)

(R,S+S,S mixture)

From 7.7 g of nitrocompound (VII), 0.3 g Pd/C, 6.5 g of p-methoxybenzaldehyde and 30 mg of $PtO_2$, 0.6 g of the product as sulphate was obtained.

NMR: (as above) d; 7.28 (d,2H), 7.14-6.36 (m,11H), 5.12 (bs,1H), 4.52 (m,1H), 4.22 (s,2H), 3.72 (2d,6H), 3.4-2.5 (m,incl. DMSO), 1.0 (d,3H).

Example 7

1-(4-hydroxy-3-p-methoxybenzylamino-phenyl)-2-(1-methyl-2-phenyl-ethylamino)-ethanol (X)

From 2.5 g of nitrocompound (VII), 260 mg of the product as sulphate was obtained.

NMR: (as above) d; 7.4-6.4 (m,12H), 5.15 (s,1H), 4.59 (bs,1H), 4.22 (s,2H), 3.70 (s,3H), 3.2-2.5 (m,incl DMSO), 1.0 (dd,3H).

Table 1

Summary of the compounds according to the invention (X)

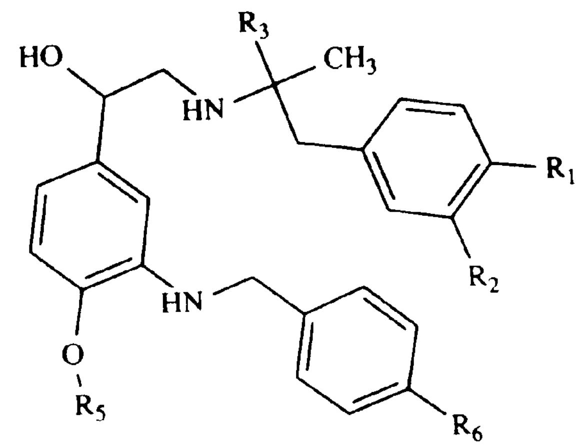

| Compound acc to Example No. | Prepared from intermediate compound of the formula | $R_1$ | $R_2$ | $R_3$ | $R_5$ | $R_6$ | Stereochemistry |
|---|---|---|---|---|---|---|---|
| 1 | VIII | $-OCH_2O-$ | | H | H | $OCH_3$ | R,S |
| 1 | VIII | $-OCH_2O-$ | | H | H | $OCH_3$ | R,R |
| 1 | VIII | $-OCH_2O-$ | | H | H | $OCH_3$ | S,R |
| 1 | VIII | $-OCH_2O-$ | | H | H | $OCH_3$ | S,S |
| 2 | VIII | $-OCH_2O-$ | | H | H | $OCH_3$ | R,S+S,R (racemate) |
| 2 | VII | $-OCH_2O-$ | | H | H | $OCH_3$ | R,R+S,S (racemate) |
| 3 | VII | $-OCH_2O-$ | | H | H | H | R,S+S,R+R,R+S,S mixture |
| 4 | VII | $-OCH_3$ | $-OCH_3$ | H | H | $OCH_3$ | R,S+S,S mixture |
| 5 | VII | $-OCH_2O-$ | | $CH_3$ | H | $OCH_3$ | R/S (racemate) |
| 6 | VII | $-OCH_3$ | -H | H | H | $OCH_3$ | R,S+S,S mixture |
| 7 | VII | H | H | H | H | $OCH_3$ | R,S+S,S mixture |

Table 2

Starting compounds of the formula (VI)

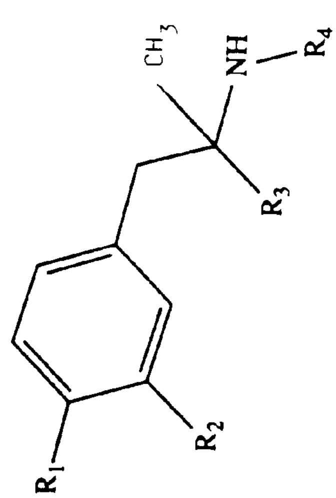

| Compound No. | Method of Preparation | $R_1$ | $R_2$ | $R_3$ | $R_4$ | Stereo-isomer(s) |
|---|---|---|---|---|---|---|
| S1 | C | $-O-CH_2-O-$ | | H | $CH(CH_3)Ph$ | S,S |
| S2 | C | $-O-CH_2-O-$ | | H | $CH(CH_3)Ph$ | R,R |
| S3 | C | $OCH_3$ | H | H | $CH(CH_3)Ph$ | S,S |
| S4 | D | $-O-CH_2-O-$ | | H | H | R/S |
| S5 | D | $-O-CH_2-O-$ | | $CH_3$ | H | - |
| S6 | D | $OCH_3$ | $OCH_3$ | H | H | R/S |
| S7 | D | H | H | H | H | R/S |
| S8 | C | H | H | H | $CH(CH_3)Ph$ | S,S |

In the following specific examples for the preparation of the compounds listed in table 2 are given:

Compound S8

N-1-(S)-phenethyl-1-phenyl-2-(S)-propaneamine

13.4 g (0.1 mole) of phenylacetone and 10.9 g of (S)-phenethylamine were refluxed in 200 ml benzene in a Dean-Stark apparatus for 3 hours. After evaporation of the solvent the residue was dissolved in ethanol. Rayney-Nickel was added and hydrogenation was carried out overnight at 50 psi. The product was purified

by recrystallization of the hydrochloride from acetone or ethanol. Yield 8.1 g (as base).
NMR: ($CDCl_3$, TMS as ref.) d; 7.33-7.06(m,10H), 3,92(1,1H), 2.87(dd,1H), 2.75(sextett, 1H), 2,48(dd,1H), 1.29(d,3H), 0.90(d,3H).

Compound S3

N-1-(S)-phenethyl-1-(4-methoxyphenyl)-2-(S)-propanamine

Prepared as above. From 16.4 g of the ketone and 10.9 g of (S)-1-phenethylamine was obtained 12.5 g of product after chromatography on a silica column ($CHCl_3$/methanol, 10/2).

Compound S1

N-1(S)-phenethyl-1-(1,3-benzodioxol-4-yl)-2(S)-propanamine and the corresponding R,R-isomer were prepared analogously from (1,3-methylendioxy)phenyl-acetone.

Compound S5

1-(3,4-methylenedioxyphenyl)-2-methyl-2-(R/S)-aminopropane

A solution of 360 g (2.4 mole) of piperonal in 684 g (7.7 mole) of 2-nitropropane was added to 2.4 mole of freshly prepared sodiummethoxide in methanol (360 ml) at 15°C. After stirring at room temperature overnight 480 ml of 5N sulfuric acid was added. The reaction mixture was then poured into water. The organic phase was treated with sodiumsulfite and recrystallized from toluene/hexane. Yield 247 g.

To a solution of 247 g in benzene was added 141 g of thionylchloride in 250 ml of benzene. After refluxing for 4 hours the solvent and excess thionylchloride were evaporated leaving an oily residue which was recrystallized from isopropyl alcohol. Yield 140 g.

26 g (0.1 mole) of this compound was dissolved in 200 ml of dioxane. 0.3 g of Pd/$BaSO_4$ was added followed by hydrogenation at 1 atm at room temperature. Filtration and evaporation gave an oily residue which was dissolved in ethanol (150 ml). 1 g of 10% Pd/C was added. Hydrogenation at 50 psi overnight gave after filtration and evaporation the product. Recrystallization gave 11.5 g of product. NMR: (hydrochloride in $CDCl_3$, ref TMS) d; 8.45 ($-NH_3$), 6.8-6.6 (aromatic), 5.9 ($OCH_2O$), 2.9 ($-CH_2-$), 1.4 ($-CH_3$).

Compound S6

1-(3,4-dimethoxyphenyl)-2-(R/S)-aminopropane

20 g (0.12 mole) of 3,4-dimethoxy benzaldehyde was dissolved in 100 ml of nitromethane. 3 ml of butylamine was added. Reflux in a Dean-Stark apparatus for 2 hours followed by evaporation gave a brown oil. Recrystallization from isopropylalcohol gave 16.6 g.

16.4 g (0.07 mole) of the nitrostyrene was added dropwise to 8 g of lithiumaluminium hydride in 1 l of dry ether. Stirring was continued overnight. After workup a yellowish oil was obtained. The sulphate was prepared from an ether/ethanol solution. Yield 10.4 g (sulphate).

Compound S4

1-(3,4-methylenedioxyphenyl)-2-(R/S)-aminopropane

Prepared according to Beng T Wo et al, J Med Chem 13 26 (1970).

Compound S7

Similarly 1-phenyl-2-(R/S)-aminopropane was prepared.

Table 3

Intermediates of the formula (VII)

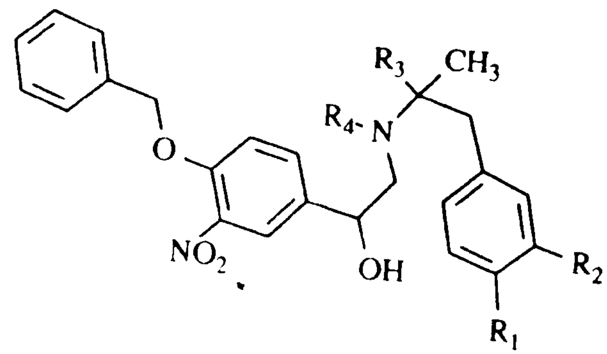

(VII)

| Compound No. | Method of Preparation | $R_1$ | $R_2$ | $R_3$ | $R_4$ | Stereoisomer(s) |
|---|---|---|---|---|---|---|
| I1 | A | $-OCH_2O-$ | | H | $CH(CH_3)Ph$ | R,S,S |
| I2 | " | $-OCH_2O-$ | | H | " | S,S,S |
| I3 | " | $-OCH_2O-$ | | H | " | R,R,R |
| I4 | " | $-OCH_2O-$ | | H | " | S,R,R |
| I5 | " | $OCH_3$ | H | H | $CH(CH_3)Ph$ | R,S,S + S,S,S mixture |
| I6 | " | H | H | H | $CH(CH_3)Ph$ | R,S,S + S,S,S mixture |
| I7 | B | $-OCH_2O-$ | | H | H | R,S + S,R racemate |
| I8 | B | $-OCH_2O-$ | | H | H | R,R + S,S racemate |
| I9 | B | $OCH_3$ | $OCH_3$ | H | H | R,S + S,R + R,R + S,S mixture |
| I10 | B | $-OCH_2O-$ | | $CH_3$ | H | R/S racemate |

In the following specific examples for the preparation of the compounds listed in table 3 are given:

General procedure for the preparation of compounds of the formula (VII), where $R_4$=H

4-benzyloxy-3-nitrostyreneoxide was refluxed with a suitable amine in methanol overnight or longer. Products were purified by chromatography on a silica column with chloroform as eluent.

1-(4-benzyloxy-3-nitrophenyl)-2-(1-methyl-2-(3,4-methylenedioxyphenyl)-ethylamino)-ethanol (R,S + S,R racemate and R,R + S,S racemate)

Described under example 2 above.

Compound I9

1-(4-benzyloxy-3-nitrophenyl)-2-(1-methyl-2-(3,4-dimethoxyphenyl)-ethylamino)-ethanol (R,R R,S S,S and S,R isomeric mixture)

From 4.3 g of the amine and 5.4 g of epoxide 5.2 g of the product as an oil was obtained.

General procedure for the preparation of a compound of the formula (VII), where $R_4$=CH($CH_3$)Ph

Amine VI was heated under a stream of dry nitrogen with 4-benzyloxy-3-nitrostyreneoxide at 150°C overnight. The crude product was purified by chromatography on a silica column with chloroform as eluent.

Compound I5

1-(4-benzyloxy-3-nitrophenyl)-2-(N-methylbenzyl-N-(1-methoxyphenyl)-2-propyl-amino)-ethanol (R,S,S + S,S,S isomeric mixture)

From 5.1 g of an amine of the formula (VI) and 5.4 g of epoxide 7.7 g of oil was obtained.
NMR: ($CDCl_3$ and TMS as reference) d; 7.86-6.71 (m,12H), 5.25(s,1H), 5.23(s,1H), 4.58(m,1/2 H), 4.37(m,1/2 H), 4.10(m,2H), 3.77(s,1.5H), 3.75(s,1.5H), 3.78(m,1/2 H), 3.25-2.40(m, ), 2.15 (m ), 16(dd,3H), 1.0(dd, 3H).

Compound I6

1-(4-benzyloxy-3-nitrophenyl)-2-(N-methylbenzyl-N-1-phenyl-2-propyl-amino)-ethanol

From 4.8 g of an amine of the formula (VI) and 5.4 g of epoxide 2.5 g of an oily product was obtained.

Compound I10

1-(4-benzyloxy-3-nitrophenyl)-2-(1,1-dimethyl-1-(3,4-methylenedioxyphenyl)-ethylamino)-ethanol (racemate)

From 4.8 g of amine VI and 5.4 g of epoxide 7.4 g of the product was obtained.
NMR: ($CDCl_3$ and TMS as reference) d; 7.85(d,1H), 7.52-6.52(m, H), 5.91(s,2H), 5.22(s,2H), 4.56(dd,1H), 2.91(dd,1H), 2.58(s,2H), 2.56(m,1H), 1.04(2s,6H).

Compounds I1, I2, I3 and I4

1-(4-benzyloxy-3-nitrophenyl)-2-(1-methyl-2-(3,4-methylenedioxyphenyl)-ethylamino)-ethanol

The preparation of the R,R,R-, R,S,S-, S,S,S- and S,R,R-isomers is described above under example 1.
Also the preparation of the compounds in the following Table 4 is described above under example 1.

Table 4

Intermediates of the formula (VIII)

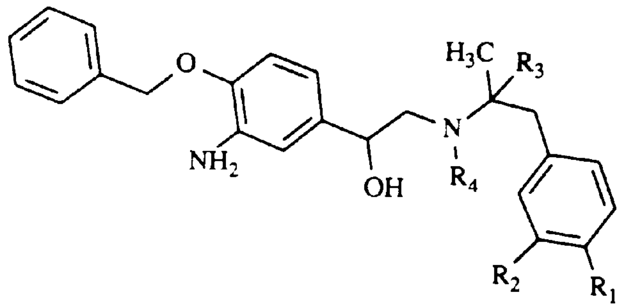

VIII

| Compound | $R_1$ | $R_2$ | $R_3$ | $R_4$ | Stereochemistry |
|---|---|---|---|---|---|
| M1 | $-OCH_2O-$ | | H | $CH(CH_3)Ph$ | R,R,R |
| M2 | $-OCH_2O-$ | | H | - " - | S,R,R |
| M3 | $-OCH_2O-$ | | H | - " - | R,S,S |
| M4 | $-OCH_2O-$ | | H | - " - | S,S,S |

The following examples illustrate how the compounds of the invention can be incorporated in pharmaceutical compositions:

Example P1. Aerosol for inhalation

| | | |
|---|---|---|
| Compound according to Example 1 or 2 | | 0.75 g |
| Sorbitan trioleate | | 0.20 g |
| Frigen[R] 11/12/114 | ad | 100.0 g |

Example P2. Tablets

Each tablet contains:

| | |
|---|---|
| Compound according to Example 1 | 6.0 mg |
| Maize starch | 78.0 mg |
| Lactose | 125.0 mg |
| Povidone | 7.0 mg |
| Microcrystalline cellulose | 13.0 mg |
| Magnesium stearate | 1.6 mg |

Example P3. Suppositories

Each suppository contains:

| | | |
|---|---|---|
| Compound according to Example 1 | | 6.0 mg |
| Ascorbyl palmitate | | 1.0 mg |
| Suppository base (Imhausen H) | ad | 2.000.0 mg |

Example P4. Syrup

| | | |
|---|---|---|
| Compound according to Example 1 | | 0.060 g |
| Liquid glucose | | 30.0 g |
| Sucrose | | 50.0 g |
| Ascorbic acid | | 0.1 g |
| Sodium pyrosulfite | | 0.01 g |
| Disodium edetate | | 0.01 g |
| Orange essence | | 0.025 g |
| Certified colour | | 0.015 g |
| Purified water | ad | 100.0 ml |

Example P5. Inhalation solution

| | | |
|---|---|---|
| Compound according to Example 1 | | 0.75 g |
| Sodium pyrosulfite | | 0.10 g |
| Disodium edetate | | 0.10 g |
| Sodium chloride | | 0.85 g |
| Purified water | ad | 100.0 ml |

Example P6. Solution for rectal administration (Rectal vials)

| | | |
|---|---|---|
| Compound according to Example 1 | | 6.0 mg |
| Sodium pyrosulfite | | 1.5 mg |
| Disodium edetate | | 0.3 mg |
| Sterile water | ad | 3.0 ml |

Example P7. Sublingual tablets

| | |
|---|---|
| Compound according to Example 1 | 3.0 mg |
| Lactose | 83.0 mg |
| Sucrose | 87.0 mg |
| Acicia | 10.0 mg |
| Talc | 5.0 mg |
| Magnesium stearate | 1.0 mg |

Example P8. Drops

| | | |
|---|---|---|
| Compound according to Example 1 | | 0.60 g |
| Ascorbic acid | | 1.00 g |
| Sodium pyrosulfite | | 0.10 g |
| Disodium edetate | | 0.10 g |
| Liquid glucose | | 50.00 g |
| Absolute alcohol | | 10.00 g |
| Purified water | ad | 100.0 ml |

Example P9

A compound according to Example 1 with the stereochemistry R,S is micronized to a particle size suitable for inhalation therapy. The substance is filled into a powder inhaler, Turbuhaler$^R$.

The typical daily dose of the active substance varies within a wide range and will depend on various factors such as for example the individual requirement of each patient, the route of administration and the disease. In general, oral and parenteral dosages will be in the range of 0.5 to 15 mg per day of active substance. The active substance may also be formed into pharmaceutical preparations containing a combination of the active compound with Ipratropium bromide or Budesonide.

Pharmacological evaluation

1. The duration of the bronchodilating activity studied in live guinea pigs.

A. Method

Concious guinea pigs were used in the study. The compounds according to Examples 1 and 2, salmeterol and terbutaline, were given by inhalation or by tracheal instillation whereafter the animals were challenged in a histamine mist after different time intervals.

a. Inhalation

The guinea pigs were placed in plastic boxes and the test compounds delivered by continuous flow nebulization during 15 minutes.

b. Tracheal instillation

The animals were lightly anaesthetized by inhalation of Efrane. The test compounds were instilled by use of a thin cannula to the tracheal bifurcation. Controlled animals were instilled with saline.

c. The provocation procedure

After the administrations, according to one of the mentioned routes, the animals were placed in transparent plastic cages and challenged with aerosolized histamine. The mist was generated in a nebulizer from a solution containing 0.5-1.0x$10^{-3}$ mol/l of histamine in 3% glycerol. This concentration produced a defined dyspnotic breathing, appearing in control animals within 2-3 minutes. This phase was usally indicated by observation, sometimes also a "blinded trial" or recorded by use of a strain-gauge belt (see 0.A.T. Olsson in Acta Allergologica 26, 438, 1971). An increasing amount of spasmolytic drug gives then rise to a dose-dependent prolongation of the ability of the animals to resist this histamine spasm. As soon as a dyspnotic breathing was established the guinea pigs were removed from the histamine milieu since the same animals have to be exposed to histamine not only after 30 minutes but also at 3, 6 and 9 hours after drug administration.

In some of the experiments the animals were challenged with methacholine instead of histamine to exclude the eventuality of an antihistaminic effect of the drugs tested.

B. Results

The β-receptor agonists salmeterol and terbutaline are both in use in the inhalation therapy and were therefore chosen as reference compounds in this study, where the new β-agonists according to this invention were evaluated with respect to the duration of action of their bronchodilating activities.

Generally, the initial potencies of the new compound according to Examples 1 and 2 are about the same as for terbutaline but approximately ten times less than for salmeterol. The duration of activity, calculated from an equipotent level, is, however, prolonged for the new compounds of the present invention and for salmeterol when compared with terbutaline. In comparison with salmeterol, the duration of the effect of the new compounds seems even to be somewhat more extended.

The compounds presented in Table 5 are 1-(4-hydroxy)-3-(p-methoxybenzyl-amino)phenyl-2-(1-methyl-2-(3,4-methylenedioxyphenyl)ethyl)amino)ethanols either in the form of the racemic mixture or as one of its isomers.

TABLE 5

| Compounds (given intra-tracheally in mol/kg) | The ability of the drugs to produce a delay (in minutes) in the appearance of dyspnotic breathing in histamine 0.5 and 6 hours after the administration | |
|---|---|---|
| | 0.5 h | 6 h |
| (R,S+S,R): $1x10^{-8}$ | 7 | 6 |
| (R,S+S,S): $1x10^{-8}$ | 6 | 5 |
| (R,R+S,R): $1x10^{-7}$ | 7 | 3 |
| (R,R): $5x10^{-8}$ | 7 | 3 |
| (R,S): $5x10^{-9}$ | 6 | 5 |
| Terbutaline (ref) $1x10^{-7}$ | 7 | 2 |
| Saline (control) | $\leq 2$ | $\leq 2$ |

The amounts instilled produce about equieffective protection (6 to 7 minutes) against a histamine provocation 30 minutes after the administration of the test compound. The degree of protection 6 hours after the instillation of the test compound indicates the duration of the bronchospasmolytic activity.

II. Study of the bronchospasmolytic and heart-stimulating effect in anaesthetized guinea pigs

The aim of this study was to compare the β-receptor agonists according to the present invention with salmeterol and terbutaline in their ability to inhibit a respiratory spasm in narcotized guinea pigs after intravenous injections or inhalation. In addition, the influence of the test compounds on the heart-rate was evaluated.

Methods

a. Intravenous administration

Guinea pigs of either sex were used in the study. The anaesthesia was produced by intravenous pentobarbital, The trachea was cannulated and the animals articificially ventilated by a constant volume respiratory. The intratracheal pressure (ITP) was measured by a pressure transducer. An increase in ITP, above the basic pressure level, indicating a respiratory spasm, was elicited by histamine infused in a jugular vein during 20 sec. every 7 minutes and in amounts producing a peak-ITP in the range 4-6 kPa. To trace cardiovascular effects from used drugs the blood pressure was recorded from a carotide artery via a pressure transducer. The pulse

wave, indicating the rate, was triggering a tachograph recording the heart frequency.

The test compounds were injected 3 minutes before a histamine infusion and the decrease in percent of the intratracheal pressure (ITP) was taken as a measure of the bronchospasmolytic activity. The influence on heart-rate was evaluated after cumultative injections; a new injection was given when the preceding one had produced a steady state in the rate.

b. Administration by inhalation

The test compounds were nebulized and the mist produced was led to the animal through the respirator and tracheal cannula. Inhalation continued for 15 mintues for each concentration of the test compounds. The bronchodilating effect on heart-rate was calculated as percent reduction in ITP. The effect on heart-rate was determined after a cumultative increase in the concentrations of the nebulized solutions. Effect measurements were made at steady state, and three different concentrations were used.

B. Results

a. Respiratory effects

The three used β-receptor agonists, the compound according to Example 2, salmeterol and terbutaline all showed a dose-related inhibition of the histamine-elicited bronchospasm after injection as well as after inhalation. Salmeterol was the most active and the potency at least ten times higher than for the other two drugs when injected and about five times when inhaled. Injection of $5x10^{-10}$ mol/kg of salmeterol, $5x10^{-9}$ mol/kg of the compound according to Example 2 or $1x10^{-8}$ mol/kg of terbutaline gives about 50 per cent reduction of ITP.

b. Effect on heart-rate

The effect on heart-rate was dose dependent and seems primarily to depend on a direct acitivity of the test compounds rather than as a result of a change in blood pressure. It was possible to demonstrate a dose related influence on the heart rate, not only after intravenous injection, but also after inhalation. When injected, salmeterol and terbutaline increased the heart-rate more than the compound of the present invention, and a dose of $1\cdot10^{-7}$ mol/kg of salmeterol and terbutaline increase the heart-rate by about 30 beats/min. To reach the same increase in heart-rate with the compounds of the present invention an at least 10 times higher dose had to be given. Inhalation of $5\cdot10^{-4}$ mol/l of salmeterol increased the heart-rate by 15 beats/min. The same increase in heart-rate was produced with $5\cdot10^{-3}$ mol/l by the compounds of the present invention, while the effect of terbutaline lies in between.

The relationship between the effect on the respiratory system and heart is the same for the compound according to Example 2 and salmeterol, when the drugs are injected. However, this relation does not seem to be the same after inhalation. In this case the compound according to Example 2 shows a more reduced effect on heart rate compared with salmeterol.

Conclusion

The compounds according to the invention are thus active during a longer period of time than e.g. terbutaline. In addition they have less effect on the heart than salmeterol.

The best mode of carrying out the invention known at present is to use the compound 1-(R)-(4-hydroxy)-3-(p-methoxybenzylamino)phenyl-2-[(1-methyl-1(S)-3,4-(methylenedioxyphenyl)ethyl)amino]ethanol.

## Claims

**Claims for the following Contracting States : AT, BE, CH, DE, DK, FR, GB, IT, LI, LU, NL, SE**

1. A compound of the formula (X)

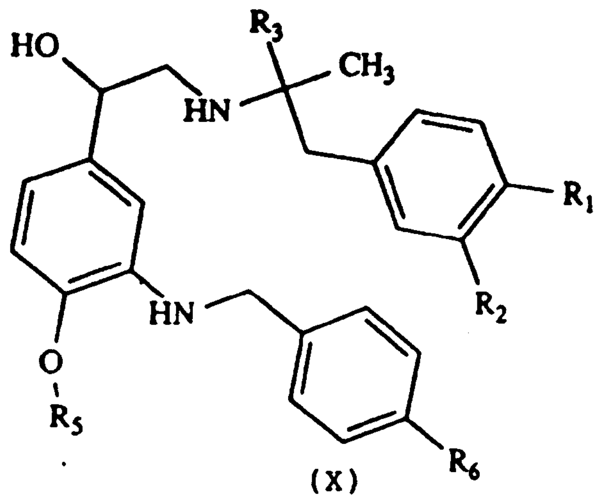

(X)

and pharmaceutically acceptable salts thereof, wherein
$R_1$ and $R_2$ together form the chain -O-$CH_2$-O- or -O-$CF_2$-O-;
$R_3$ is H or $CH_3$;
$R_5$ is H or -CO-$R_7$;
$R_6$ is H, OH, $OCH_3$, $OC_2H_5$ or halogen;
$R_7$ is a straight or branched alkyl group with 1-16 carbon atoms, aryl, -$NHCH_3$ or -$N(CH_3)_2$.

2. A compound according to claim 1, wherein $R_6$ representing a halogen is fluor, chlor or brom and $R_7$ representing an aryl is a group with 3-8 carbon atoms, preferably 6 carbon atoms.

3. A compound according to claim 1 in the form of a substantially pure optical isomer.

4. A compound according to claim 1, wherein $R_1$ and $R_2$ together form the chain -O-$CH_2$-O-, $R_3$ is H, $R_6$ is H and $R_6$ is $OCH_3$.

5. A compound according to claim 1, which is the mixture of R,S and S,S isomers of 1-(4-hydroxy)-3-(p-methoxybenzylamino)phenyl-2-[(1-methyl-2-(3,4-methylenedioxyphenyl)-ethyl)-amino)ethanol or a pharmaceutically acceptable salt thereof.

6. A compound according to claim 1, which is the racemate of the R,S and S,R isomers of 1-(4-hydroxy)-3-(p-methoxybenzylamino)phenyl-2-[(1-methyl-2-(3,4-methylenedioxyphenyl)-ethyl)-amino]ethanol or a pharmaceutically acceptable salt thereof.

7. A compound according to claim 1, which is the isomer 1(R)-(4-hydroxy)-3-(p-methoxybenzylamino)phenyl-2-[(1-(S)-methyl-2--(3,4-methylenedioxyphenyl)-ethyl)-amino]ethanol or a pharmaceutically acceptable salt thereof.

8. The hydrochloride of the compound according to claim 7.

9. A process for the preparation of a compound of the formula (X) according to claim 1, whereby 4-benzyloxy-3-nitro-styreneoxide of the formula (V)

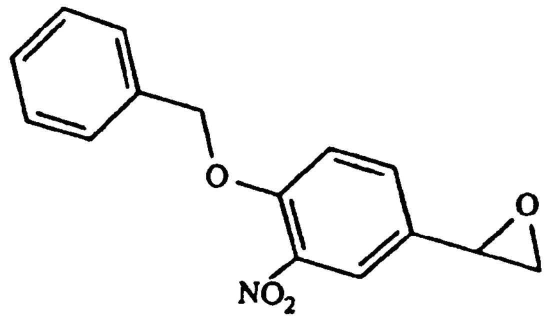

(V)

is reacted with a compound of the formula (VI)

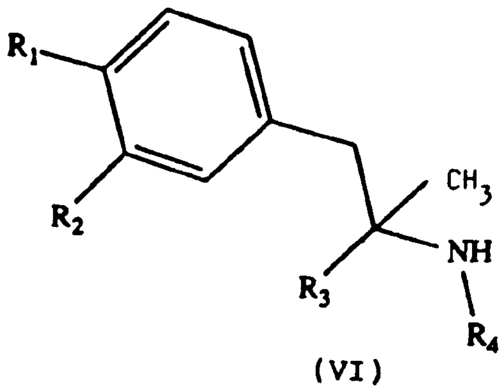

(VI)

wherein $R_1$, $R_2$ and $R_3$ are as defined above in claim 1 and $R_4$ is hydrogen or

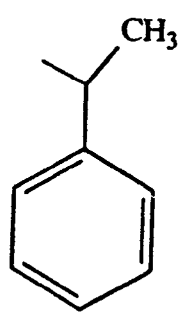

to give a compound of the formula (VII)

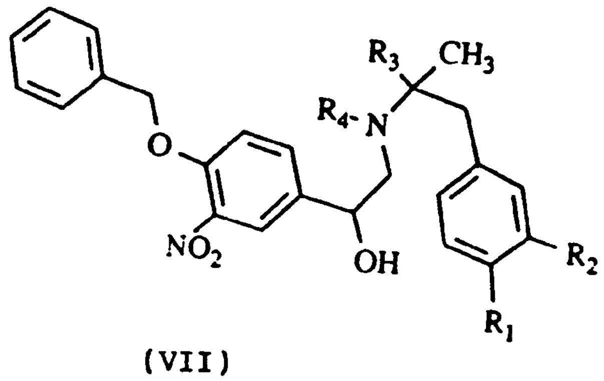

(VII)

wherein $R_1$, $R_2$, $R_3$ and $R_4$ are as defined above, whereafter the compound of the formula (VII) is reacted with hydrogen and a suitable catalyst and with

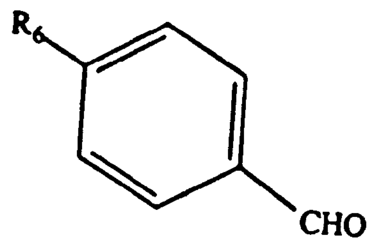

wherein $R_6$ is as defined above to yield after hydrogenation a compound of the formula (X) as a pure isomer, a racemate or a mixture of isomers.

10. Pharmaceutical preparations containing a compound according to claim 1 together with a pharmaceutically acceptable carrier.

11. Use of a compound according to claim 1 in the manufacture of a preparation with prolonged broncho-spasmolytic activity.

**Claims for the following Contracting States : ES, GR**

1. A process for the preparation of a compound of the formula (X)

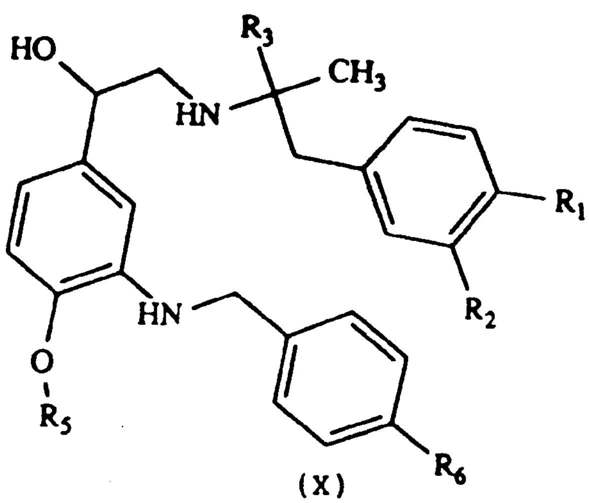

(X)

and pharmaceutically acceptable salts thereof, wherein
$R_1$ and $R_2$ together form the chain -O-$CH_2$-O- or -O-$CF_2$-O-;

$R_3$ is H or $CH_3$;
$R_5$ is H or $-CO-R_7$;
$R_6$ is H, OH, $OCH_3$, $OC_2H_5$ or halogen;
$R_7$ is a straight or branched alkyl group with 1-16 carbon atoms, aryl, $-NHCH_3$ or $-N(CH_3)_2$, whereby 4-benzyloxy-3-nitro-styreneoxide of the formula (V)

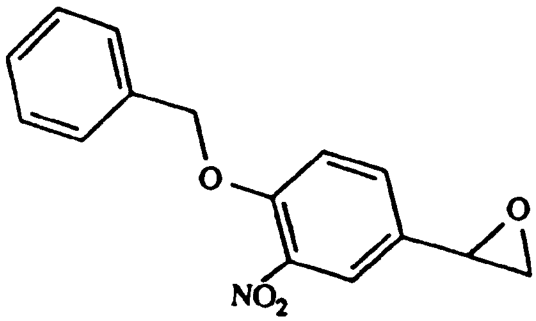

(V)

is reacted with a compound of the formula (VI)

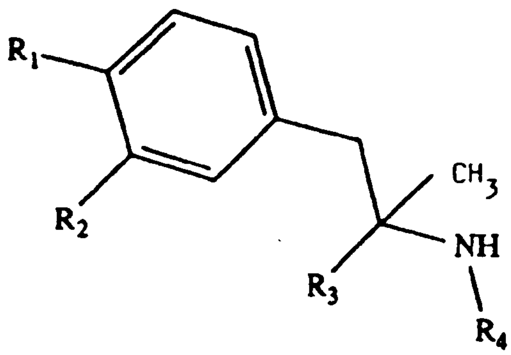

(VI)

wherein $R_1$, $R_2$ and $R_3$ are as defined above in claim 1 and $R_4$ is hydrogen or

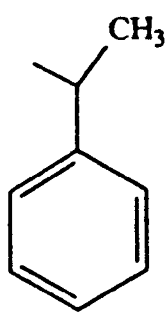

to give a compound of the formula (VII)

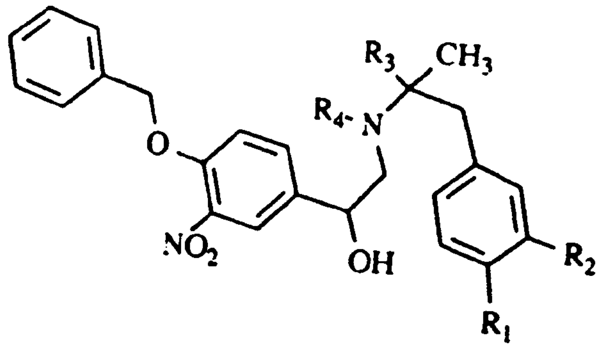

(VII)

wherein $R_1$, $R_2$, $R_3$ and $R_4$ are as defined above, whereafter the compound of the formula (VII) is reacted with hydrogen and a suitable catalyst and with

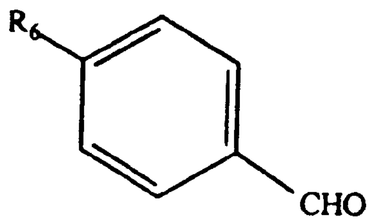

wherein $R_6$ is as defined above to yield after hydrogenation a compound of the formula (X) as a pure isomer, a racemate or a mixture of isomers.

2. A process according to claim 1, wherein in the compound of the formula X $R_6$ representing a halogen is fluor, chlor or brom and $R_7$ representing an aryl is a group with 3-8 carbon atoms, preferably 6 carbon atoms.

3. A process according to claim 1 wherein a compound of the formula X in the form of a substantially pure optical isomer is obtained.

4. A process according to claim 1, wherein a compound of the formula X where $R_1$ and $R_2$ together form the chain $-O-CH_2-O-$, $R_3$ is H, $R_6$ is H and $R_6$ is $OCH_3$ is obtained.

5. A process according to claim 1, wherein the mixture of R,S and S,S isomers of 1-(4-hydroxy)-3-(p-methoxybenzylamino)phenyl-2-[(1-methyl-2-(3,4-methylenedioxyphenyl)-ethyl)-amino]ethanol or a pharmaceutically acceptable salt thereof is obtained.

6. A process according to claim 1, wherein the racemate of the R,S and S,R isomers of 1-(4-hydroxy)-3-(p-methoxybenzylamino)phenyl-2-[(1-methyl-2-(3,4-methylenedioxyphenyl)-ethyl)-amino]ethanol or a pharmaceutically acceptable salt thereof is obtained.

7. A process according to claim 1, wherein the isomer 1-(R)-(4-hydroxy)-3-(p-methoxybenzylamino)phenyl-2-[(1-(S)-methyl-2--(3,4-methylenedioxyphenyl)-ethyl)-amino]ethanol or a pharmaceutically acceptable salt thereof is obtained.

8. A process according to claim 7 wherein the compound is in the form of its hydrochloride.

9. A process for the preparation of pharmaceutical preparations containing a compound of the formula X, wherein said compound is mixed with a pharmaceutically acceptable carrier.

10. Use of a compound of the formula X according to claim 1 in the manufacture of a preparation with prolonged broncho-spasmolytic activity.

## Patentansprüche

### Patentansprüche für folgende Vertragsstaaten : AT, BE, CH, DE, DK, FR, GB, IT, LI, LU, NL, SE

1. Verbindung der Formel (X)

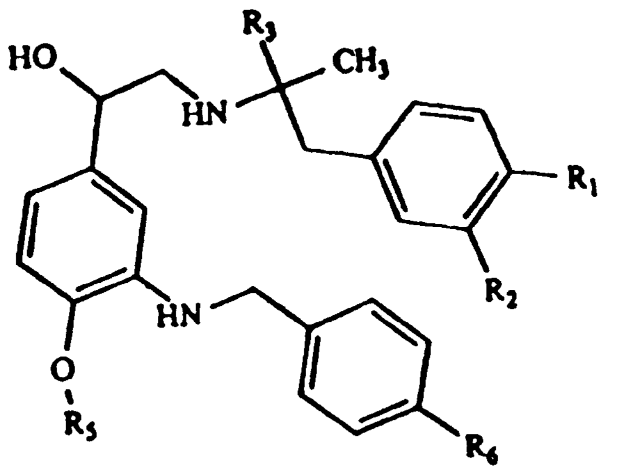

(X)

und pharmazeutisch annehmbare Salze hievon, worin $R_1$ und $R_2$ zusammen die Kette $-O-CH_2-O-$ oder $-O-CF_2-O-$ bilden; $R_3$ die Bedeutung H oder $CH_3$ hat; $R_5$ H oder $-CO-R_7$ darstellt; $R_6$ H, OH, $OCH_3$, $OC_2H_5$ oder Halogen ist; und $R_7$ eine gerad- oder verzweigtkettige Alkylgruppe mit 1 bis 16 Kohlenstoffatomen, Aryl, $-NHCH_3$ oder $-N(CH_3)_2$ bedeutet.

2. Verbindung nach Anspruch 1, worin Halogen darstellendes $R_5$ Fluor, Chlor oder Brom ist, und Aryl darstellendes $R_7$ eine Gruppe mit 3 bis 8 Kohlenstoffatomen, vorzugsweise 6 Kohlenstoffatomen, ist.

3. Verbindung nach Anspruch 1, in Form eines im wesentlichen reinen optischen Isomers.

4. Verbindung nach Anspruch 1, worin $R_1$ und $R_2$ zusammen die Kette $-O-CH_2-O-$ bilden, $R_3$ die Bedeutung H hat, $R_5$ **d**arstellt und $R_5$ $OCH_3$ ist.

5. Verbindung nach Anspruch 1, welche die Mischung von R,S- und S,S-Isomeren von 1-(4-Hydroxy)-3-(p-methoxybenzylamino)-phenyl-2-[(1-methyl-2-(3,4-methylendioxyphenyl)-ethyl)-amino]-ethanol oder ein pharmazeutisch annehmbares Salz hievon ist.

6. Verbindung nach Anspruch 1, welche das Racemat der R,S- und S, R-Isomere von 1-(4-Hydroxy)-3-(p-methoxybenzylamino)-phenyl-2-[(1-methyl-2-(3,4-methylendioxyphenyl)-ethyl)-amino]-ethanol oder ein pharmazeutisch annehmbares Salz hievon ist.

7. Verbindung nach Anspruch 1, welche das Isomer 1-(R)-(4-Hydroxy)-3-(p-methoxybenzylamino)-phenyl-2-[(1-(S)-methyl-2-(3,4-methylendioxyphenyl)-ethyl)-amino]-ethanol oder ein pharmazeutisch annehmbares Salz hievon ist.

8. Hydrochlorid der Verbindung nach Anspruch 7.

9. Verfahren zur Herstellung einer Verbindung der Formel (X) nach Anspruch 1, bei welchem 4-Benzyloxy-3-nitrostyroloxid der Formel (V)

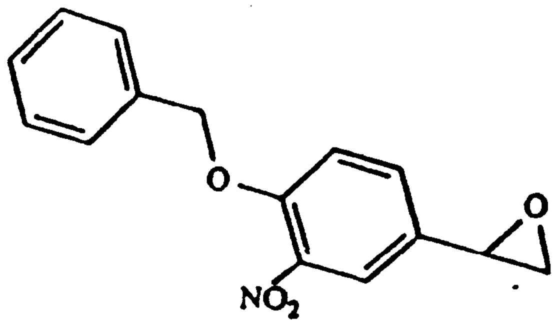

(V)

mit einer Verbindung der Formel (VI)

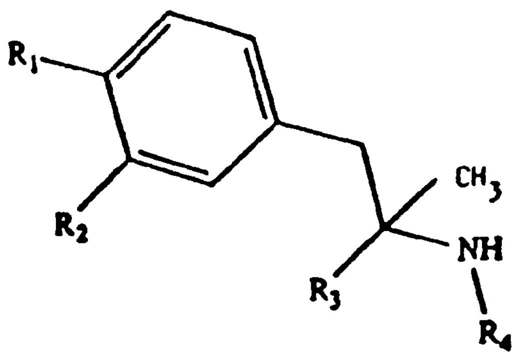

(VI),

worin $R_1$, $R_2$ und $R_3$ wie oben in Anspruch 1 definiert sind, und $R_4$ Wasserstoff oder

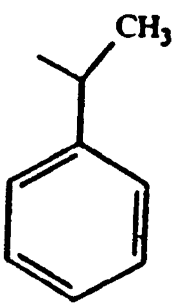

bedeutet, umgesetzt wird, wobei eine Verbindung der Formel (VII)

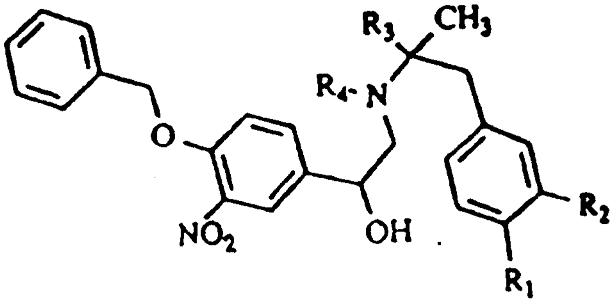

(VII),

worin $R_1$, $R_2$, $R_3$ und $R_4$ wie oben definiert sind, erhalten wird, wonach die Verbindung der Formel (VII) mit Wasserstoff und einem geeigneten Katalysator und mit

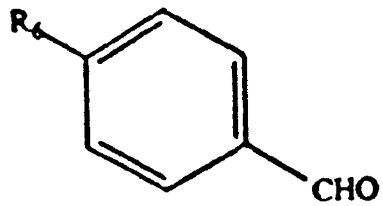

worin $R_6$ wie oben definiert ist, umgesetzt wird, wobei nach Hydrierung eine Verbindung der Formel (X) als reines Isomer, Racemat oder Mischung von Isomeren erhalten wird.

**10.** Pharmazeutische Zubereitungen, welche eine Verbindung nach Anspruch 1 zusammen mit einem pharmazeutisch annehmbaren Träger enthalten.

**11.** Verwendung einer Verbindung nach Anspruch 1, bei der Herstellung einer Zubereitung mit verlängerter bronchospasmolytischer Wirksamkeit.

**Patentansprüche für folgende Vertraggsstaaten : ES, GR**

**1.** Verfahren zur Herstellung einer Verbindung der Formel (X)

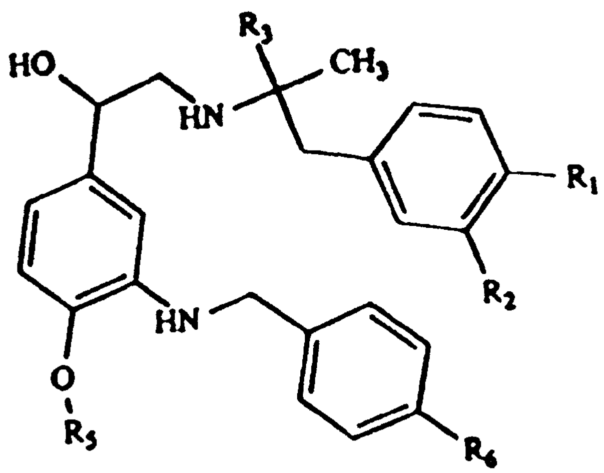

**(X)**

und pharmazeutisch annehmbarer Salze hievon, worin $R_1$ und $R_2$ zusammen die Kette $-O-CH_2-O-$ oder $-O-CF_2-O-$ bilden; $R_3$ die Bedeutung H oder $CH_3$ hat; $R_6$ H oder $-CO-R_7$ darstellt; $R_6$ H, OH, $OCH_3$, $OC_2H_5$ oder Halogen ist; und $R_7$ eine gerad- oder verzweigtkettige Alkylgruppe mit 1 bis 16 Kohlenstoffatomen, Aryl, $-NHCH_3$ oder $-N(CH_3)_2$ bedeutet, bei welchem Verfahren 4-Benzyloxy-3-nitrostyroloxid der Formel (V)

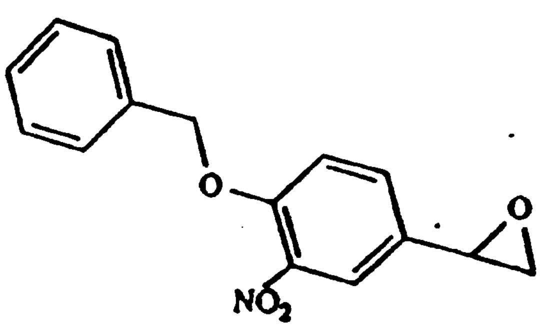

**(V)**

mit einer Verbindung der Formel (VI)

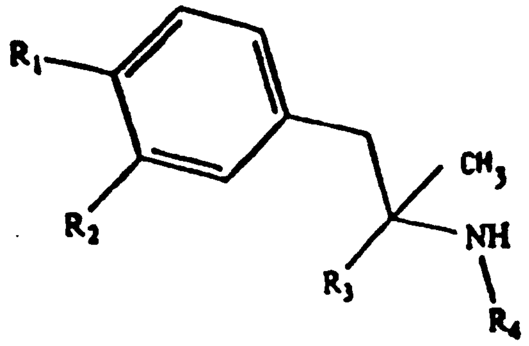

(VI),

worin $R_1$, $R_2$ und $R_3$ wie oben in Anspruch 1 definiert sind, und $R_4$ Wasserstoff oder

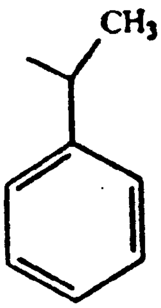

bedeutet, umgesetzt wird, wobei eine Verbindung der Formel (VII)

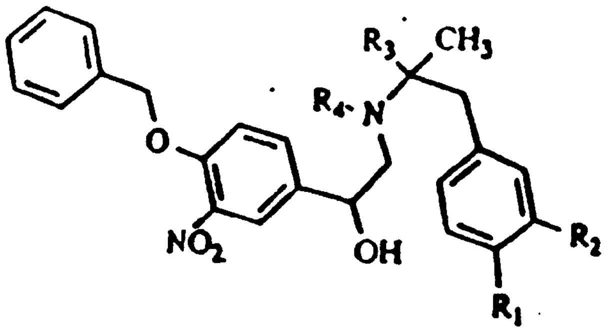

(VII),

worin $R_1$, $R_2$, $R_3$ und $R_4$ wie oben definiert sind, erhalten wird, wonach die Verbindung der Formel (VII) mit Wasserstoff und einem geeigneten Katalysator und mit

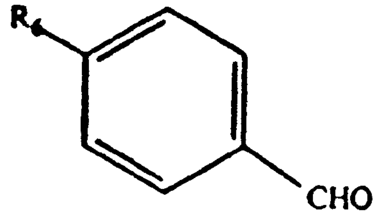

,

worin $R_6$ wie oben definiert ist, umgesetzt wird, wobei nach Hydrierung eine Verbindung der Formel (X) als reines Isomer, Racemat oder Mischung von Isomeren erhalten wird.

2. Verfahren nach Anspruch 1, bei welchem in der Verbindung der Formel (X) Halogen darstellendes $R_6$ Fluor, Chlor oder Brom ist, und Aryl darstellendes $R_7$ eine Gruppe mit 3 bis 8 Kohlenstoffatomen, vorzugsweise 6 Kohlenstoffatomen, ist.

3. Verfahren nach Anspruch 1, bei welchem eine Verbindung der Formel (X) in Form eines im wesentlichen reinen optischen Isomers erhalten wird.

4. Verfahren nach Anspruch 1, bei welchem eine Verbindung der Formel (X), worin $R_1$ und $R_2$ zusammen die Kette $-O-CH_2-O-$ bilden, $R_3$ die Bedeutung H hat, $R_5$ H darstellt und $R_5$ $OCH_3$ ist, erhalten wird.

5. Verfahren nach Anspruch 1, bei welchem die Mischung von R,S- und S,S-Isomeren von 1-(4-Hydroxy)-3-(p-methoxybenzylamino)-phenyl-2-[(1-methyl-2-(3,4-methylendioxyphenyl)-ethyl)-amino]-ethanol oder ein pharmazeutisch annehmbares Salz hievon erhalten wird.

6. Verfahren nach Anspruch 1, bei welchem das Racemat der R,Sund S,R-Isomere von 1-(4-Hydroxy)-3-(p-methoxybenzylamino)-phenyl-2-[(1-methyl-2-(3,4-methylendioxyphenyl)-ethyl)-amino]-ethanol oder ein pharmazeutisch annehmbares Salz hievon erhalten wird.

7. Verfahren nach Anspruch 1, bei welchem das Isomer 1-(R)-(4-Hydroxy)-3-(p-methoxybenzylamino)-phenyl-2-[(1-(S)-methyl-2-(3,4-methylendioxyphenyl)-ethyl)-amino]-ethanol oder ein pharmazeutisch annehmbares Salz hievon erhalten wird.

8. Verfahren nach Anspruch 7, bei welchem die Verbindung in Form ihres Hydrochlorids vorliegt.

9. Verfahren zur Herstellung pharmazeutischer Zubereitungen, die eine Verbindung der Formel (X) enthalten, bei welchem die Verbindung mit einem pharmazeutisch annehmbaren Träger gemischt wird.

10. Verwendung einer Verbindung der Formel (X) nach Anspruch 1, bei der Herstellung einer Zubereitung mit verlängerter bronchospasmolytischer Wirksamkeit.

## Revendications

**Revendications pour les Etats contractants suivants : AT, BE, CH, DE, DK, FR, GB, IT, LI, LU, NL, SE**

1. Composé de formule (X)

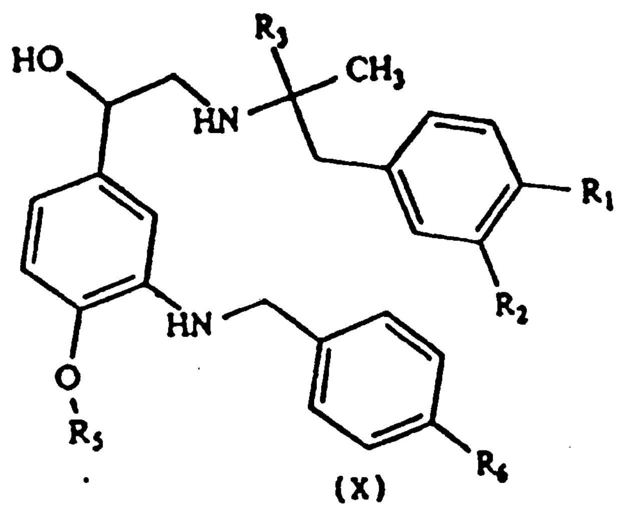

(X)

et sels pharmaceutiquement acceptables de ce dernier, formule dans laquelle $R_1$ et $R_2$ forment ensemble la chaîne
$-O-CH_2-O-$ ou $-O-CF_2-O-$;
$R_3$ est H ou $CH_3$;
$R_5$ est H ou $-CO-R_7$;
$R_6$ est H, OH, $OCH_3$, $OC_2H_5$ ou un halogène;

$R_7$ est un groupe alkyle linéaire ou ramifié comportant 1-16 atomes de carbone, un aryle, $-NHCH_3$ ou $-N(CH_3)_2$.

2. Composé selon la revendication 1, dans lequel $R_6$ représentant un halogène est un fluor, un chlore ou un brome, et $R_7$ représentant un groupe aryle est un groupe comportant 3-8 atomes de carbone, de préférence 6 atomes de carbone.

3. Composé selon la revendication 1, sous forme d'un isomère essentiellement optiquement pur.

4. Composé selon la revendication 1, dans lequel $R_1$ et $R_2$ forment ensemble la chaîne $-O-CH_2-O-$, $R_3$ est H, $R_5$ est H et $R_6$ est $OCH_3$.

5. Composé selon la revendication 1, qui est le mélange des isomères R,S et S,S du 1-(4-hydroxy)-3-(p-méthoxybenzylamino)phényl-2-[(1-méthyl-2-(3,4-méthylènedioxyphényl)éthyl)amino]éthanol, ou un sel pharmaceutiquement acceptable de ce mélange.

6. Composé selon la revendication 1, qui est le racémate des isomères R,S et S,R du 1-(4-hydroxy)-3-(p-méthoxybenzylamino)phényl-2-[(1-méthyl-2-(3,4-méthylènedioxyphényl)éthyl)amino]éthanol, ou un sel pharmaceutiquement acceptable de ce racémate.

7. Composé selon la revendication 1, qui est l'isomère 1-(R)-(4-hydroxy)-3-(p-méthoxybenzylamino)phényl-2-[(1-(S)-méthyl-2-(3,4-méthylènedioxyphényl)ethyl)amino]éthanol, ou un sel pharmaceutiquement acceptable de ce dernier.

8. Chlorhydrate du composé selon la revendication 7.

9. Procédé de préparation d'un composé de formule (X) selon la revendication 1, dans lequel on fait réagir l'oxyde de 4-benzyloxy-3-nitrostyrène de formule (V)

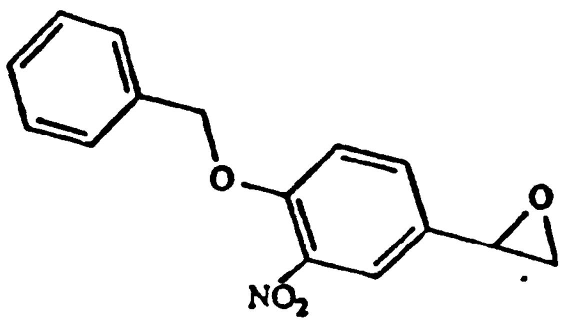

(V) .

avec un composé de formule (VI)

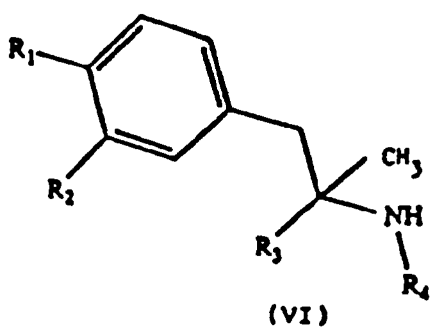

(VI)

dans laquelle $R_1$, $R_2$ et $R_3$ sont tels que définis ci-dessus dans la revendication 1, et $R_4$ est un hydrogène, ou

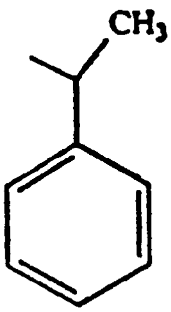

pour obtenir un composé de formule (VII)

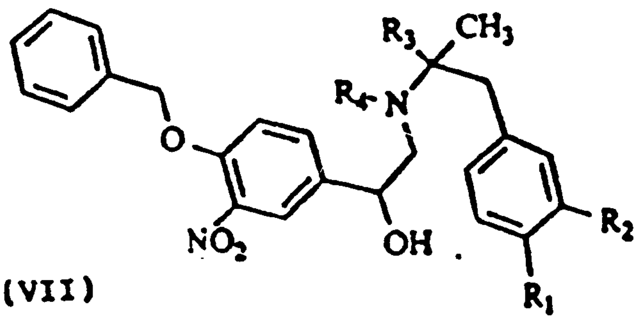

(VII)

dans laquelle $R_1$, $R_2$, $R_3$ et $R_4$ sont tels que définis ci-dessus, puis on fait réagir le composé de formule (VII) avec de l'hydrogène et un catalyseur convenable et avec

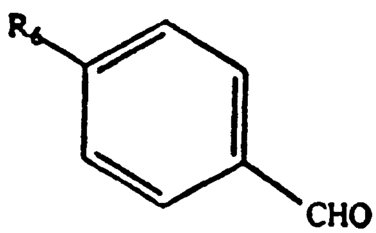

où $R_6$ est tel que défini ci-dessus dans la revendication 6, pour obtenir, après hydrogénation, un composé de formule (X) sous forme d'isomère pur, de racémate, ou de mélange d'isomères.

10. Préparations pharmaceutiques contenant un composé selon la revendication 1 avec un véhicule pharmaceutiquement acceptable.

11. Utillsation d'un composé selon la revendication 1 pour fabriquer une préparation ayant une activité anti-bronchospasmodique prolongée.

**Revendications pour les Etats contractants suivants : ES, GR**

1. Procédé de preparation d'un composé de formule (X)

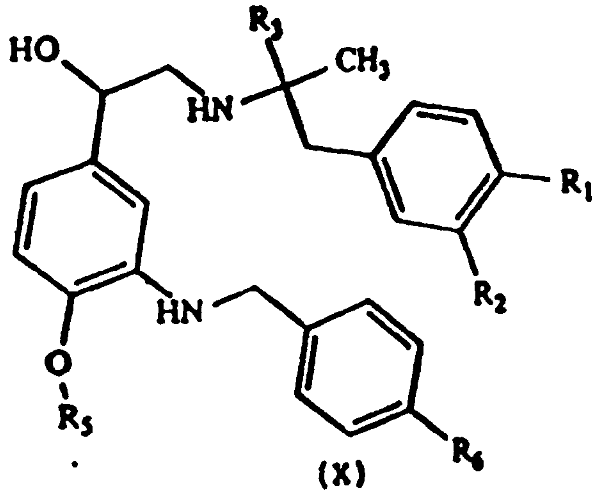

(X)

et de sels pharmaceutiquement acceptables de ce dernier, formule dans laquelle $R_1$ et $R_2$ forment ensemble la chaîne $-O-CH_2-O-$ ou $-O-CF_2-O-$;
$R_3$ est H ou $CH_3$;
$R_5$ est H ou $-CO-R_7$;
$R_6$ est H, OH, $OCH_3$, $OC_2H_5$ ou un halogène;
$R_7$ est un groupe alkyle linéaire ou ramifié comportant 1-16 atomes de carbone, aryle, $-NHCH_3$ ou $-N(CH_3)_2$,
procédé par lequel on fait réagir l'oxyde de 4-benzyloxy-3-nitrostyrène de formule (V)

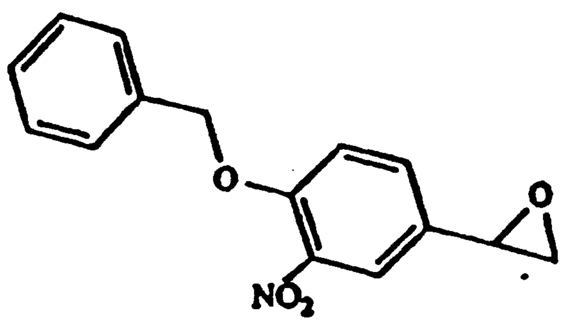

(V)

avec un composé de formule (VI)

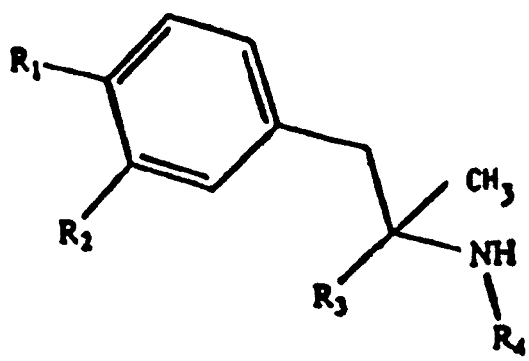

(VI)

dans laquelle $R_1$, $R_2$ et $R_3$ sont tels que définis ci-dessus dans la revendication 1, et $R_4$ est un hydrogène, ou

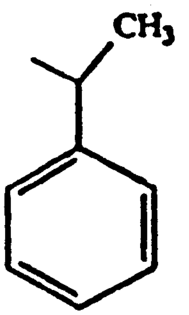

pour obtenir un composé de formule (VII)

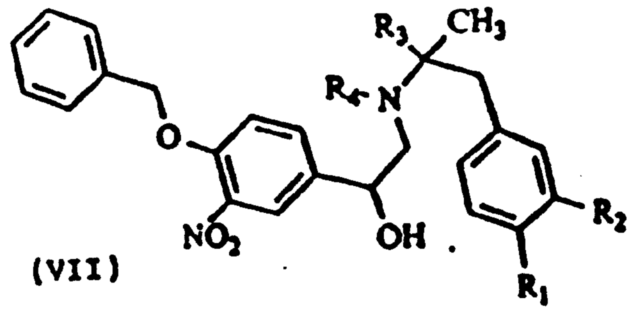

(VII)

dans laquelle $R_1$, $R_2$, $R_3$ et $R_4$ sont tels que définis ci-dessus, puis on fait réagir le composé de formule (VII) avec de l'hydrogène et un catalyseur convenable et avec

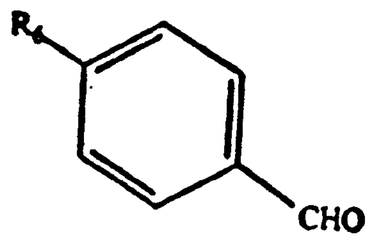

où $R_6$ est tel que défini ci-dessus pour obtenir, après hydrogénation, un composé de formule (X) sous forme d'isomère pur, de racémate, ou de mélange d'isomères.

2. Procédé selon la revendication 1, dans lequel, dans le composé de formule (X), $R_6$ représentant un halogène est un fluor, un chlore ou un brome, et $R_7$ représentant un groupe aryle est un groupe comportant 3-8 atomes de carbone, de préférence 6 atomes de carbone.

3. Procéde selon la revendication 1, dans lequel on obtient un composé de formule (X) est sous forme d'un isomère essentiellement optiquement pur.

4. Procédé selon la revendication 1, dans lequel on obtient un composé de formule (X) dans laquelle $R_1$ et $R_2$ forment ensemble la chaîne $-O-CH_2-O-$, $R_3$ est H, $R_6$ est H et $R_6$ est $OCH_3$.

5. Procédé selon la revendication 1, dans lequel on obtient le mélange des isomères R,S et S,S du 1-(4-hydroxy)-3-(p-méthoxybenzylamino)phényl-2-[(1-méthyl-2-(3,4-méthylènedioxyphényl)éthyl)amino]ét hanol, ou un sel pharmaceutiquement acceptable de ce mélange.

6. Procédé selon la revendication 1, dans lequel on obtient le racémate des isomères R,S et S,R du 1-(4-hydroxy)-3-(p-methoxybenzylamino)phényl-2-[(1-méthyl-2-(3,4-méthylènedioxyphényl)éthyl)amino]ét hanol, ou un sel pharmaceutiquement acceptable de ce racémate.

7. Procédé selon la revendication 1, dans lequel on obtient l'isomère 1-(R)-(4-hydroxy)-3-(p-méthoxyben-

zylamino)phényl-2-[(1-(S)-méthyl-2-(3,4-méthylènedioxyphenyl)éthyl)amino]éthanol, ou un sel pharmaceutiquement acceptable de ce dernier.

8. Procédé selon la revendication 7, dans lequel le composé est sous sa forme chlorhydrate.

9. Procédé de préparation de préparations pharmaceutiques contenant un composé de formule (X), dans lequel ledit composé est mélangé avec un véhicule pharmaceutiquement acceptable.

10. Utilisation d'un composé de formule X selon la revendication 1 pour fabriquer une préparation ayant une activité antibronchospasmodique prolongée.